(19) Europäisches Patentamt
European Patent Office
Office européen des brevets

(11) Numéro de publication : **0 136 353**
**B1**

(12) # FASCICULE DE BREVET EUROPÉEN

(45) Date de publication du fascicule du brevet : **05.04.89**

(51) Int. Cl.⁴ : **C 12 N 5/00**

(21) Numéro de dépôt : **84901363.6**

(22) Date de dépôt : **23.03.84**

(86) Numéro de dépôt international : **PCT/FR 84/00080**

(87) Numéro de publication internationale : **WO/8403710 (27.09.84 Gazette 84/23)**

(54) **MILIEU DE CULTURE DE CELLULES ANIMALES SANS SERUM ET PROCEDES DE CULTURE PRIMAIRE ET D'OBTENTION DE LIGNEES CELLULAIRES UTILISANT CE MILIEU.**

(30) Priorité : **24.03.83 FR 8304843**

(43) Date de publication de la demande : **10.04.85 Bulletin 85/15**

(45) Mention de la délivrance du brevet : **05.04.89 Bulletin 89/14**

(84) Etats contractants désignés : **BE CH DE FR GB LI LU NL SE**

(56) Documents cités :
**EP--A-- 0 060 565**
**EP--A-- 0 076 647**
**FR--A-- 2 059 471**
**FR--A-- 2 464 994**
**GB--A-- 2 016 044**
**GB--A-- 2 064 543**
**Hoppe-Seyler's Z. Physiol. Chem. vol. 361, published in 1980. J.H. Wissler et al.: "Leucocyte-derived protein hormones for tissue repair (lympho-mono and leucokines): large-scale production, isolation and properties of cell-derived cytotaxins, sytokinesins, cytotoxins and mitogens", pages 351-352, see page 351 right-hand column, lines 11-26**

(73) Titulaire : **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur : **CHESSEBEUF, Martine**
**9, rue Lorraine**
**F-21000 Dijon (FR)**
Inventeur : **PADIEU, Prudent**
**6, rue Jehan de Vienne Chevigny Saint-Sauveur**
**F-21800 Quetigny (FR)**

(74) Mandataire : **Ayache, Monique et al**
**CABINET PLASSERAUD 84, rue d'Amsterdam**
**F-75009 Paris (FR)**

**EP 0 136 353 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention concerne les milieux de culture de cellules animales.

Elle a plus particulièrement pour objet un milieu synthétique de culture de cellules animales, notamment de cellules épithéliales ou de cellules myélomateuses et leurs hybrides de type hybridome, ne renfermant pas de sérum et convenant tant à la culture primaire de cellules animales, qu'à l'obtention de lignées cellulaires à partir de cette culture primaire ou d'une autre. Elle a également pour objet un procédé de culture primaire et un procédé d'obtention d'une lignée de cellules animales utilisant un tel milieu.

Depuis un certain nombre d'années, des recherches intensives ont été effectuées afin de mettre au point un milieu de culture permettant d'obtenir des lignées de cellules hépatiques présentant les caractéristiques des hépatocytes parenchymateux, afin notamment d'élucider les mécanismes régulateurs qui contrôlent la prolifération et la différenciation des cellules hépatiques.

Jusqu'à présent deux types de cultures de cellules hépatiques ont été largement développés à partir de cellules obtenues par dissociation du tissu hépatique au moyen de collagénase ou de trypsine.

On savait que la digestion du foie par la collagénase fournit des hépatocytes parenchymateux non proliférants qui peuvent être maintenus plusieurs jours, voire plusieurs semaines, soit dans un milieu de culture additionné de sérum (Serum Supplemented Medium : SSM), soit dans un milieu de culture sans sérum (Serum Free Medium : SFM). Ces cellules parenchymateuses manifestent différentes fonctions du foie et remplacent par conséquent avantageusement les homogénats ou coupes tissulaires de foie. Toutefois, la sénescence inévitable de ces hépatocytes ne permet pas des études de longue durée, ce qui a incité les chercheurs à mettre au point des cultures proliférantes de cellules hépatiques épithéliales. De telles cultures ont été, dans un premier temps, obtenues dans des milieux additionnés de sérum (SSM), après digestion du tissu hépatique par la trypsine ou à partir de cultures primaires initiées par la collagénase. Bien que la croissance de cellules hépatiques épithéliales normales présente en général des difficultés, notamment en raison d'un développement trop important de cellules fibroblastiques, on a pu montrer que les cultures proliférantes de cellules hépatiques épithéliales possèdent des caractéristiques des cellules hépatiques parenchymateuses. Toutefois, l'addition de sérum au milieu de culture introduit dans celui-ci un très grand nombre de composants dont notamment des produits finals et des effecteurs, c'est-à-dire régulateurs, des métabolismes du foie qui compliquent, voire même rendent impossible, l'utilisation de lignées cellulaires obtenues dans des milieux additionnés de sérum, en particulier dans le cas de certaines études telles que celle relatives à la biosynthèse du cholestérol, des stérols biliaires neutres et des acides biliaires.

Pour remédier, au moins en partie, aux différents inconvénients présentés par les lignées cellulaires obtenues dans des milieux de culture additionnés de sérum, différents auteurs ont proposé de remplacer le sérum par des hormones et des facteurs de croissance tels qu'en particulier l'insuline et l'« épidermal growth factor », dont la présence était jusqu'à présent considérée comme indispensable au développement de lignées cellulaires.

Or, la dépendance de nombreux processus métaboliques du foie vis-à-vis des hormones est bien connue. En conséquence, la présence d'hormones dans le milieu de culture conduit également à des modifications ou à des perturbations, voire même dans certains cas à l'impossibilité d'effectuer des études fiables sur des lignées de cellules hépatiques obtenues dans des milieux de culture additionnés d'hormones.

Dans le cas de l'utilisation industrielle de lignées de cellules épithéliales établies et de lignées myélomateuses et leurs hybrides de type hybridome, la présence de sérum ou de composants inconnus ou mal définis conduit à une grande difficulté, voire même à une impossibilité, de purifier le ou les produits biologiques cellulaires à des fins d'administration à l'homme. De plus, la non-définition de la composition du milieu conduit à l'interdiction d'une telle utilisation humaine de ces produits biologiques par les autorités sanitaires internationales et nationales (OMS, FDA, Ministère de la Santé, etc.).

Le but de l'invention est de fournir un milieu de culture de cellules animales ne présentant pas les inconvénients des milieux de culture connus.

Or, on a constaté que de façon surprenante et contre toute attente, il est possible d'effectuer tant des cultures primaires que des cultures secondaires dans un milieu ne comportant ni sérum, et dans la plupart des cas ni hormones, ni facteurs de croissance, en l'additionnant en des proportions bien définies, de certains acides gras ou de leurs esters en présence d'un biopolymère lipophile en une quantité capable de permettre la dissolution des acides gras ou de leurs esters ajoutés.

L'un des objets de l'invention est donc un milieu de culture du type susindiqué ne comprenant ni sérum, et dans la plupart des cas ni hormones, ni facteurs de croissance, caractérisé en çe qu'il est additionné, en des proportions bien définies, de certains acides gras ou de leurs esters, en présence de biopolymère lipophile en une quantité capable de permettre la dissolution de la quantité d'acides gras ou de leurs esters ajoutée.

Dans son esprit le plus général l'invention fournit donc un milieu de culture de cellules animales dépourvu de sérum, caractérisé par l'utilisation d'un milieu de base contenant les nutriments indispensables à la croissance des cellules animales et d'acides gras déterminés ou leurs esters, à des concentrations relatives et à une concentration globale propres à assurer la croissance desdites cellules,

ces concentrations ne devant pas dépasser un seuil au-delà duquel ces acides gras ou leurs esters deviendraient toxiques à l'égard des cultures cellulaires. Il va par ailleurs de soi que les acides gras utilisés, ou leurs esters, doivent avoir une concentration supérieure à un seuil minimum pour autoriser la croissance des cellules animales.

L'invention a plus particulièrement pour objet un milieu de culture de cellules animales, sans sérum, caractérisée en ce qu'il est constitué :

a) d'un milieu synthétique de base conçu pour la culture de cellules animales :

b) de 0,05 à 30 μeq/l d'un mélange des six acides gras : acide palmitique, acide cis-palmitoléique, acide stéarique, acide cis-oléique, acide cis-linoléique et acide cis-linolénique présents chacun sous la forme de l'acide libre ou de l'un de ses esters, dans les proportions suivantes, rapportées à l'acide gras libre :

— de 0,0155 à 24,0 μmole/l d'acide palmitique ;
— de 0,0058 à 9,0 μmole/l d'acide cis-palmitoléique ;
— de 0,0014 à 2,0 μmole/l d'acide stéarique ;
— de 0,0067 à 10,0 μmole/l d'acide cis-oléique ;
— de 0,0178 à 27,0 μmole/l d'acide cis-linoléique ; et
— de 0,0028 à 4,0 μmole/l d'acide cis-linolénique,

adsorbés sur

c) au moins un biopolymère lipophile présent en une quantité capable d'assurer la dissolution des acides gras ou de leurs esters présents dans le milieu.

Le biopolymère lipophile est de préférence constitué par un polysaccharide polymoléculaire contenant des motifs d'α-glucopyrannose, connu sous la dénomination commune de dextrane, de préférence en une quantité de 0,5 à 200 mg/l de milieu de culture ou par de l'albumine délipidée ou non, de préférence en une quantité de 2 à 6 g/l de milieu de culture.

Des résultats équivalents étant obtenus avec l'albumine délipidée d'une part et avec l'albumine non délipidée d'autre part, il semble que les lipides contenus au départ dans l'albumine n'interviennent pas dans le processus de prolifération des cellules. Dans ces conditions, compte tenu du coût moindre de l'albumine non délipidée, elle est tout particulièrement préférée.

Selon un autre mode de réalisation, l'invention a pour objet un milieu de culture de cellules animales, sans sérum, caractérisé en ce qu'il est constitué :

a) d'un milieu synthétique de base conçu pour la culture de cellules animales ;

b) de 0,05 à 30 μeq/l d'un mélange des six acides gras : acide palmitique, acide cis-palmitoléique, acide stéarique, acide cis-oléique, acide cis-linoléique et acide cis-linolénique présents sous la forme de l'acide libre ou de l'un de ses esters, dans les proportions suivantes, rapportées à l'acide gras libre :

— de 0,0155 à 24,0 μmole/l d'acide palmitique ;
— de 0,0058 à 9,0 μmole/l d'acide cis-palmitoléique ;
— de 0,0014 à 2,0 μmole/l d'acide stéarique ;
— de 0,0067 à 10,0 μmole/l d'acide cis-oléique ;
— de 0,0178 à 27,0 μmole/l d'acide cis-linoléique ; et
— de 0,0028 à 4,0 μmole/l d'acide cis-linolénique,

adsorbés sur

c) au moins un dextrane présent en une quantité capable d'assurer la dissolution des acides gras ou de leurs esters présents dans le milieu ; et éventuellement d'hormones et/ou de facteurs de croissance.

Ce milieu de culture contient de préférence de 0,5 à 200 mg/l de dextrane.

Le dextrane utilisé est de préférence le dextrane homogène de poids moléculaire 2 000 000.

Quelle que soit la dilution des acides gras utilisés ou de leurs esters dans le milieu de culture, il s'est avéré avantageux de les utiliser dans les proportions molaires suivantes :

| | |
|---|---|
| — acide palmitique | 31,0 % |
| — acide cis-palmitoléique | 11,6 % |
| — acide stéarique | 2,8 % |
| — acide cis-oléique | 13,4 % |
| — acide cis-linoléique | 35,6 % |
| — acide cis-linolénique | 5,6 % |

Selon un mode préféré de réalisation de l'invention, le milieu comprend en outre une concentration non cytotoxique, pour toute la durée de passage de la culture, d'un antibiotique, de préférence à large spectre. De préférence on utilise, en tant qu'antibiotique, de la gentamycine à raison de 25 à 100 mg/l.

Selon un autre mode de réalisation de l'invention, les six acides gras sont présents dans le milieu à l'état d'acides libres.

En tant que milieu synthétique de base on peut avantageusement avoir recours à un milieu synthétique utilisé de façon habituelle pour la culture des cellules animales, tel que notamment :

— les milieux de Ham,
— le milieu Waymouth MB 752/1,
— les milieux RPMI 1629 et 1640,
— le milieu de Eagle,
— les milieux de Eagle modifiés,
— le milieu E de Williams,
— le milieu 199 et les milieux dérivés de types MEM et MEMα.

Des résultats particulièrement bons ont été obtenus avec le milieu commercialisé sous la référence « Ham F 10 medium » par la société Gibco, Grand Island, New York, EUA, dont la composition sera indiquée plus loin et le milieu E de Williams caractérisé par une plus grande richesse en vitamines.

Si le milieu de culture contient l'un ou plusieurs des acides gras mentionnés plus haut, ou leurs esters, il devra en être tenu compte dans le calcul de la concentration globale en acides gras.

Dans certains cas particuliers tels que notamment culture de cellules myélomateuses et de leurs hybrides de type hybridome, il peut s'avérer utile, voire nécessaire, d'ajouter au milieu de culture des hormones et/ou des facteurs de croissance habituellement utilisés dans les cultures cellulaires. A titre indicatif, on peut avoir recours à des hormones et/ou facteurs de croissance choisis parmi ceux mentionnés par G. Sato et Coll. dans Anal. Biochem. (1980), 102, 235-270 et les utiliser aux doses indiquées dans cet article.

Un autre objet de l'invention est constitué par un procédé de culture primaire de cellules animales, utilisant un tel milieu.

Le procédé de culture primaire selon l'invention comprend essentiellement les étapes consistant à :

1) prélever le tissu renfermant les cellules à cultiver et le découper très finement, dans un milieu de culture de base, de préférence dans le milieu de base devant être utilisé mais ne contenant pas d'ions $Ca^{2+}$ et $Mg^{2+}$, tiédi (30-37 °C), dans des conditions aseptiques ;

2) après rinçage, de préférence dans le milieu de base devant être utilisé mais ne contenant pas d'ions $Ca^{2+}$ et $Mg^{2+}$, soumettre le tissu à digestion enzymatique.

3) transférer la suspension de cellules ainsi libérées dans un milieu de base, de préférence celui utilisé pour la culture, centrifuger en poursuivant parallèlement la digestion du tissu restant.

4) mettre le culot de centrifugation en suspension dans le milieu de culture selon l'invention contenant éventuellement un agent de surfactage du substratum de culture puis,

5) inoculer la suspension ainsi obtenue sur le substratum de culture préalablement ou simultanément surfacté,

6) incuber dans un incubateur étanche, saturé par la vapeur d'eau, en présence d'un mélange d'azote, d'oxygène et de gaz carbonique dans des proportions utilisables en biologie de la respiration cellulaire, en renouvelant périodiquement le milieu de culture,

les étapes 3) à 6) étant répétées sur les différentes fractions de cellules désirées dissociées, obtenues successivement à l'étape 2), en utilisant à chaque fois un nouveau substratum de culture.

La digestion enzymatique est effectuée au moyen d'une enzyme protéolytique telle que la collagénase ou de préférence la trypsine, éventuellement associée avec d'autres enzymes agissant sur les saccharides complexes, telles que la hyaluronidase et/ou sur les acides polynucléiques, tels que DNA-ase et RNA-ase, selon une méthode connue en soi, par exemple celle décrite par M. Chessebeuf et Coll. dans Biochimie, 56, 1365-1379 (1974).

Le volume de milieu de culture ajouté à l'étape 4) correspond en pratique au volume intérieur du récipient contenant le substratum de culture devant être utilisé à l'étape 5).

Comme substratum de culture, on peut avoir recours notamment à toute boîte habituellement utilisée pour la culture des cellules animales, telles que boîte de Pétri ou, de préférence, boîte de Cooper ou encore à des microporteurs sphériques de culture.

Le surfactage du substratum semble avoir pour rôle de permettre « l'accrochage » des cellules et favoriser ainsi leur prolifération. Il peut être effectué avant l'ensemencement, selon des méthodes usuelles, connues de l'homme du métier, par exemple au moyen de sérum, de collagène, de polylysine ou de fibronectine. Différents types de sérums, de préférence le sérum de veau fœtal ou mieux le sérum de veau nouveau-né qui est moins coûteux, peuvent être utilisés à cet effet.

Après le surfactage, le substratum étant rincé à plusieurs reprises, de préférence avec le milieu de base utilisé, avant l'ensemencement, seuls restent sur le substratum les éléments devant servir à l'« accrochage » des cellules, alors que, dans le cas du sérum, les constituants qui sont actifs dans la croissance cellulaire sont éliminés.

Le surfactage peut également être effectué simultanément à la culture, notamment grâce à la présence de fibronectine dans le milieu de culture.

Un autre objet de l'invention est un procédé d'obtention d'une lignée de cellules animales utilisant le milieu de culture selon l'invention. Ce procédé est caractérisé par le fait qu'il comprend essentiellement les étapes consistant à :

a) soumettre à digestion enzymatique le produit cellulaire d'une culture antérieure, notamment primaire, effectuée sur un substratum et ayant de préférence atteint la confluence, pour initier une ou plusieurs culture(s) secondaire(s),

b) inoculer les cellules animales obtenues sur un ou plusieurs substratums,

c) effectuer la ou les cultures(s) secondaire(s) dans le milieu de culture selon l'invention, et

d) répéter les étapes a) à c) autant de fois que nécessaire pour conserver la lignée cellulaire pendant le temps désiré.

Encore un autre objet de l'invention est un procédé d'obtention d'une lignée cellulaire utilisant le milieu de culture selon l'une des revendications 1 à 9, caractérisé en ce qu'il comprend essentiellement les étapes consistant à :

a) mettre des cellules provenant d'une culture antérieure en suspension dans le milieu de culture,

b) effectuer la culture des cellules dans ledit milieu de culture et

c) répéter les étapes a) et b) autant de fois que nécessaire pour conserver la lignée cellulaire pendant le temps désiré.

L'invention sera mieux comprise à l'aide des exemples détaillés décrits ci-dessous. Il va de soi que ces exemples ont uniquement pour but d'illustrer et de mieux expliquer l'invention, sans en limiter aucunement la portée.

Exemple 1

Culture de cellules épithéliales de foie de rat

— Milieu de culture :

Le milieu de base est obtenu à partir du milieu de Ham en poudre commercialisé sous la référence « Ham F 10 medium » par la société Gibco, Grand Island, New York, EUA. Après dissolution dans de l'eau tridistillée, il a la composition suivante :

| Composants | mg/l |
|---|---|
| L-alanine | 9,000 |
| L-arginine HCl | 211,000 |
| L-asparagine $H_2O$ | 15,010 |
| Acide L-aspartique | 13,000 |
| L-cystéine | 25,000 |
| Acide L-glutamique | 14,700 |
| L-glutamine | 146,000 |
| Glycine | 7,510 |
| L-histidine HCl $H_2O$ | 23,000 |
| L-isoleucine | 2,600 |
| L-leucine | 13,000 |
| L-lysine Hcl | 29,000 |
| L-méthionine | 4,480 |
| L-phénylalanine | 5,000 |
| L-proline | 11,500 |
| L-serine | 10,500 |
| L-thréonine | 3,570 |
| L-tryptophane | 0,600 |
| L-tyrosine (sel disodique | 2,620 |
| L-valine | 3,500 |
| Biotine | 0,024 |
| D-pantothénate de Ca | 0,715 |
| Chlorure de choline | 0,698 |
| Acide folique | 1,320 |
| L-inositol | 0,541 |
| Nicotinamide | 0,615 |
| Pyridoxine HCl | 0,206 |
| Riboflavine | 0,376 |
| Thiamine HCl | 1,000 |
| Vitamine B12 | 1,360 |
| $CaCl_2$ $2H_2O$ | 44,000 |
| $CuSO_4$ $5H_2O$ | 0.0025 |
| $FeSO_4$ $7H_2O$ | 0,8340 |
| KCl | 285,000 |
| $KH_2PO_4$ | 83,000 |
| $MgSO_4$ $7H_2O$ | 153,000 |

(Suite)

| Composants | mg/l |
|---|---|
| NaCl | 7 400,000 |
| $Na_2HPO_4$ | 153,700 |
| $NaHCO_3$ | 1 200,000 |
| $ZnSO_4$ $7H_2O$ | 0,0288 |
| Glucose | 1 100,0000 |
| Hypoxanthine | 4,680 |
| Acide lipoïque | 0,200 |
| Rouge de phénol | 1,200 |
| Pyruvate de sodium | 110,000 |
| Thymidine | 0,700 |
| Eau tridistillée qsp | 1 000 ml |

A ce milieu sont ajoutés 50 mg/l de gentamycine et les acides gras libres utilisés selon l'invention, commercialisés par la société NU CHECK PREP, ELYSIAN, MN, sont ajoutés, à une concentration totale en acides gras de 7,6 μeq/l, dans les proportions molaires suivantes :

| | |
|---|---|
| — acide palmitique | 31,0 % |
| — acide cis-palmitoléique | 11,6 % |
| — acide stéarique | 2,8 % |
| — acide cis-oléique | 13,4 % |
| — acide cis-linoléique | 35,6 % |
| — acide cis-linoléique | 5,6 % |

ces acides étant adsorbés sur 4 g/l d'albumine (n° 1 4503 de la société Sigma Saint Louis, MO, EUA).

— Culture primaire des cellules épithéliales de foie de rat :

Les foies de 8 rats nouveaux-nés, de préférence du même sexe, sont excisés et découpés finement dans des conditions aseptiques. Après deux rinçages dans du milieu « Ham F 10 medium » ne contenant toutefois pas d'ions $Ca^{2+}$ et $Mg^{2+}$, les foies finement découpés sont transférés dans un flacon de trypsinisation de 50 ml, muni d'un agitateur magnétique. On leur ajoute un volume égal d'une solution diluée 3 fois par le milieu de rinçage de trypsine 1 : 250 (c'est-à-dire de la trypsine qui hydrolyse 250 fois son poids de caséine) provenant de la société Microbiological Associates, Bethesda, MD, EUA. On agite 10 minutes à 100 T/mm, à 37 °C, puis on décante la suspension de cellules dans du milieu « Ham F 10 medium » refroidi par de la glace (2 volumes de milieu pour un volume de cellules) et on centrifuge à 30 g. Parallèlement, on renouvelle la solution de trypsinisation dans le flacon.

On répète la digestion séquentielle par la trypsine tant que le rendement de digestion se maintient (cellules épithéliales viables identifiées au microscope), c'est-à-dire en général de 6 à 9 fois.

Chaque culot de centrifugation comprenant environ $10^5$ cellules épithéliales viables est mis en suspension dans environ 4,3 ml du milieu de culture selon l'invention, décrit plus haut, et inoculé dans 1 boîte de Cooper (n° 3 009 de la société Falcon, Oxnard, CA, EUA) surfactée par du sérum. Les boîtes sont maintenues à 37 °C dans un incubateur étanche de culture (Lequeux, Paris, France) saturé par la vapeur d'eau, en présence d'un mélange azote, oxygène et gaz carbonique à 76 % : 19 % : 5 %. On change le milieu de culture tous les deux jours.

Le surfactage des boîtes s'effectue au moyen de sérum de veau fœtal ou nouveau-né par incubation une nuit à 37 °C. Après rejet du sérum, on rince deux fois avec du milieu « Ham F 10 medium » tiédi (30-37 °C), avant l'inoculation.

Peu après l'inoculation, les cellules épithéliales s'attachent sous forme de nombreux placards monocouches composés de 100 à 500 cellules chacun. La croissance de ces plages conduit à des cultures confluentes d'une population pure en cellules épithéliales de $10^6$ cellules par boîte en moins de 8 jours, en moyenne après 6 jours pour une surface de 20 $cm^2$.

Il est frappant de constater l'inaptitude du milieu de culture selon l'invention à favoriser la croissance de cellules fibroblastoïdes et endothéliales. Le milieu de culture selon l'invention conduit donc à une sélection cellulaire en favorisant une croissance privilégiée de cellules épithéliales.

Propagation cellulaire :

Lorsque les cultures primaires ont sensiblement atteint la confluence, le contenu de chaque boîte est traité par la solution de trypsine utilisée précédemment pour la digestion du foie et mis en culture secondaire pour initier deux nouvelles cultures.

Le milieu de culture selon l'invention permet d'effectuer avec succès des cultures primaires et des cultures secondaires jusqu'à un nombre de passages élevé (des résultats positifs ont encore été obtenus au bout de 80 passages).

Etude des fonctions hépatiques des cellules épithéliales de foie obtenues selon l'invention :

Pour évaluer les qualités des cellules épithéliales de foie obtenues selon l'invention, on a étudié, sur ces cellules, des fonctions caractéristiques du foie vis-à-vis du métabolisme de composés endogènes, à savoir l'inductibilité de la L-tyrosine aminotransférase par les glucocorticoïdes, la biosynthèse des acides biliaires et le métabolisme de la progestérone.

Résultats obtenus :

A. Activité de la L-tyrosine aminotransférase (TAT) et son induction ou inductibilité par les glucocorticoïdes

L'induction bien connue de l'enzyme TAT (EC 2.6.1.5) par les glucocorticoïdes in vivo a été démontrée pour la première fois en 1957 ; elle est souvent utilisée pour prouver le maintien d'une activité hépatique dans des systèmes de foie en culture.

L'activité de la TAT et son inductibilité par les glucocorticoïdes ($10^{-6}$ mole/l de dexaméthasone (dex) pendant 5 heures à 37 °C) ont été étudiées dans les lignées de cellules épithéliales hépatiques obtenues selon l'invention, en suivant la technique décrite par T.I. Diamondstone dans Anal. Biochem. 16, 395-401 (1966) et en comparant à l'induction obtenue in vivo dans du foie de rat postnatal. L'étude a été faite sur cinq lignées de cellules épithéliales de foie obtenues selon l'invention. Deux lignées (54C♂ 5FRS et 54C(D)♂ 5FRS) ont été étudiées depuis le 8ème passage jusqu'au 28ème passage, deux autres (45A♂ 6FRS et 45A(D)♂ 6FRS) depuis le 8ème ou respectivement le 14ème passage, jusqu'au 30ème passage. Le tableau I suivant montre l'évolution du taux d'induction en fonction de la progression du nombre de passages. Les taux d'induction étaient principalement compris entre 1,5 et 2,1 dans les premiers passages pour les 2 lignées 54C et 45A. Ces taux d'induction ont ensuite commencé à diminuer pour revenir à 1 vers le 15ème passage pour la lignée 54C et vers le 25ème passage pour la lignée 45A. Les lignées 54C(D) et 45A(D) ont maintenu un taux d'induction supérieur à 1 respectivement jusqu'au 25ème passage pour la première et jusqu'au 30ème passage pour la deuxième.

En conclusion, les lignées obtenues selon l'invention ont montré qu'elles avaient une activité basale de la TAT correspondant à 20 à 30 % de celle du foie in vivo dans le cas de rats du même âge, mais que le taux d'induction était d'environ 75 % de celui observé in vivo, dans les mêmes conditions.

Tableau I

Expression de l'inductibilité de la TAT par les glucocorticoïdes.

| 54C♂ 5 FRS | | 54C(D)♂ 5FRS | | 45A♂ 6FRS | | 45A(D)♂ 6FRS | |
|---|---|---|---|---|---|---|---|
| 8,7 ± 3,7 | | 7,3 ± 4,3 | | 5,6 ± 3,3 | | 6,5 ± 2,8 | |
| P n° | I/B | P n° | I/B | P n° | I/B | P n° | I/B |
| 8 | 1,5 | 8 | 1,6 | 8 | 1,7 | - | - |
| 12 | 1,5 | 12 | 1,3 | 14 | 2,0 | 14 | 2,5 |
| 15 | 1,8 | - | - | 18 | 1,2 | 18 | 1,7 |
| 19 | 0,7 | 18 | 2,1 | 21 | 1,4 | - | - |
| 25 | 1,0 | 25 | 1,2 | 24 | 1,5 | 24 | 1,7 |
| 28 | 1,1 | 28 | 0,8 | 30 | 1,1 | 30 | 1,3 |

1ère ligne : référence de la lignée cellulaire ;

2ème ligne : moyenne ± coefficient de variation de l'activité basale de la TAT pour chaque lignée cellulaire ;

3ème ligne : P n° = numéro de passage et I/B = taux d'inductibilité, c'est-à-dire activité après induction/activité basale.

L'activité spécifique de la TAT est exprimée en mU/mg de protéine. Une unité est la quantité d'enzyme formant 1 nmole de pyruvate de p-hydroxyphényle par minute à 37 °C. Chaque valeur représente la moyenne de deux essais. L'activité enzymatique et la teneur en protéines ont été déterminées en utilisant trois boîtes de culture avec monocouche de cellules soit par le témoin soit pour l'induction par la dexaméthasone (dex) ($10^{-6}$ mole/l pendant 5 heures à 37 °C). Les cellules ont été récoltées après double rinçage par 2 ml de PBS (tampon salin au phosphate) froid puis grattage avec 2 ml de PBS et ont été immédiatement séparées sous forme d'un culot de centrifugation avant entreposage à — 30 °C. Après décongélation, les cellules ont été mises en présence d'un volume total de tampon de 1 ml (phosphate de potassium 0,1 M, pH 7,6, EDTA 1 mM, pyridoxal phosphate 0,2 mM et cétoglutarate de Na 5 nM). La suspension résultante a été centrifugée à 40 000 g pendant 30 minutes avant l'étude de l'activité enzymatique.

B. Biosynthèse des acides biliaires

L'acide chénodésoxycholique et l'acide cholique sont les deux acides biliaires (BA) principaux, dits

acides biliaires primaires synthétisés par le foie de la plupart des espèces animales, tandis que dans le foie de rat il se produit une synthèse biliaire primaire particulière, la biosynthèse de l'acide α-muricholique. C'est une production spécifique des espèces murines qui correspond à une hydroxylation en position 6 β de l'acide chénodésoxycholique.

La biosynthèse des acides biliaires libres a été recherchée dans des lignées cellulaires obtenues selon l'invention.

1. Biosynthèse des acides cholique et chénodésoxycholique

Cette étude a été effectuée sur une lignée cellulaire SFM obtenue selon l'invention, au 11ème passage. Les acides biliaires ont été extraits du milieu de culture et analysés par chromatographie en phase gazeuse et par chromatographie en phase gazeuse couplée à la spectrométrie de masse. Le tracé chromatographique et l'analyse des spectres de masse repérés ont montré que la lignée étudiée au 11ème passage synthétisait, dans les conditions de l'étude, l'acide cholique et l'acide chénodésoxycholique. Les spectres de masse étaient tout-à-fait en conformité avec ceux des standards. Cette méthode a permis de calculer la quantité d'acides biliaires synthétisée, exprimée en nanogrammes par 24 heures et par mg de cellules en culture. Ce taux de synthèse était de 11,4 nanogrammes dans le cas de l'acide chénodésoxycholique libre et de 63 nanogrammes dans le cas de l'acide cholique libre.

On rappellera, pour comparaison, que dans le cas du foie de rat in vivo, il a été démontré que la synthèse totale des acides biliaires primaires (acides chénodésoxycholique et cholique) qu'ils soient libres ou conjugués, était de l'ordre de 280 nanogrammes par 24 heures et par mg de foie frais.

2. Biosynthèse de l'acide α-muricholique

Une autre lignée a été étudiée au 9ème passage en incubant avec de l'acide chénodésoxycholique radioactif marqué au carbone 14 ($^{14}C$) en position 24. Cet acide biliaire dihydroxylé a été hydroxylé en position 6, dans l'isomérie 6 β, pour donner naissance à l'acide α-muricholique marqué en position 24 par le carbone 14. Le taux d'acide α-muricholique synthétisé était de l'ordre de 100 nanogrammes par 24 heures et par mg de cellules cultivées. Cette valeur est tout-à-fait comparable à la valeur trouvée in vivo chez le rat.

Ces observations sur la biosynthèse des acides biliaires montrent d'une façon pertinente que les cellules épithéliales de foie de rat cultivées dans le milieu de culture selon l'invention sont capables de maintenir en activité les hydroxylases du cycle stéroïde qui sont des enzymes microsomales dépendantes du cytochrome P450. Ces hydroxylases qui permettent la synthèse de l'acide cholique et de l'acide chénodésoxycholique appartiennent à la classe du cytochrome P450 agissant sur les composés endogènes, tandis que la 6 β-cholanoylhydroxylase qui transforme l'acide chénodésoxycholique en acide α-muricholique est une hydroxylase à cytochrome P450 qui agit sur des composés exogènes, du type de celles qui interviennent dans la biotransformation des xénobiotiques.

Ainsi, on a mis en évidence la biosynthèse des acides biliaires par les lignées de cellules hépatiques épithéliales de foie obtenues grâce au milieu de culture selon l'invention. Cette observation n'aurait pu être faite sur des lignées cellulaires contenant du sérum. En effet, il est connu que les acides biliaires sont des inhibiteurs de leur propre biosynthèse donc, dans le cas de cultures de cellules épithéliales de foie obtenues dans un milieu contenant du sérum, normalement les acides biliaires qui sont présents dans le sérum inhibent la biosynthèse des acides biliaires par les cellules de foie.

Ainsi, grâce à la culture de cellules de foie dans le milieu selon l'invention on a pu mettre en évidence d'une façon pertinente la biosynthèse des acides biliaires. De plus, ces cellules de foie étant des cellules de foie de rat, on a pu mettre en évidence le maintien en culture d'une caractéristique spécifique du foie de rat, à savoir la synthèse de l'acide α-muricholique.

C. Métabolisme de la progestérone

Le métabolisme de la progestérone représenté plus loin de façon schématique a été vérifié sur deux lignées de cellules hépatiques épithéliales provenant de rats impubères, du 4ème au 7ème passage.

De la progestérone marquée au carbone 14 en position 4 a été incubée à un taux physiologique (62-80 nmoles/1) pendant 24 heures à la confluence.

L'extrait stéroïdien de chaque passage a été chromatographié en couche mince et la radioactivité des fractions séparées sur chaque plaque a été mesurée au moyen d'un compteur de radioactivité.

Cette étude a montré que 87 % du précurseur avaient été métabolisés en quatre fractions contenant respectivement des prégnanetriols, de la 20 α-dihydroprogestérone et des prégnanediols, des prégnanolones contenant de la progestérone résiduaire, et de la prégnanedione.

Chaque fraction obtenue par chromatographie en couche mince a été analysée et quantifiée par chromatographie en phase gazeuse associée à la spectrométrie de masse. On a ainsi pu mettre en évidence la présence de 11 métabolites représentés sur le schéma qui suit.

(Voir schéma page 9)

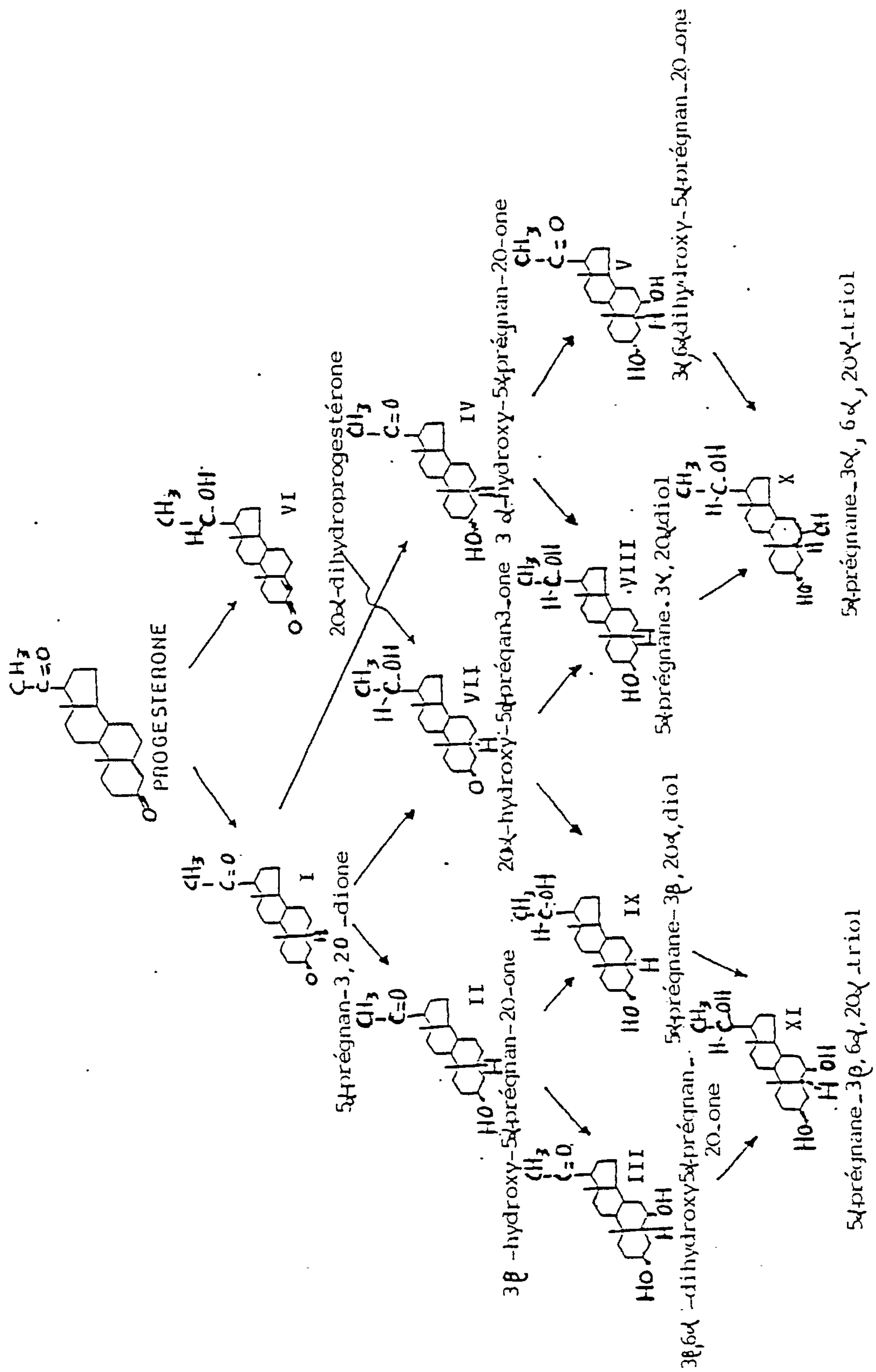

PROGESTERONE

I 5α-prégnan-3, 20 -dione

II 3β -hydroxy-5α-prégnan-20-one

III 3β,6α -dihydroxy-5α-prégnan-20-one

IV 3 α-hydroxy-5α-prégnan-20-one

V 3α,6α-dihydroxy-5α-prégnan-20-one

VI 20α-dihydroprogestérone

VII 20α-hydroxy-5α-prégnan3-one

VIII 5α-prégnane-3α, 20α-diol

IX 5α-prégnane-3β, 20α, diol

X 5α-prégnane-3α, 6α, 20α-triol

XI 5α-prégnane-3β, 6α, 20α-triol

Les caractéristiques métaboliques les plus marquantes du foie ont été trouvées dans ces cultures, à savoir :

— réduction 20 α du groupe $C_{20}$-oxo (composés VI à IX) ;

— production de prégnanetriols (composés X et XI) due à la 6 α-prégnanolone hydroxylase, enzyme qui est un « marqueur » spécifique du foie ;

— présence unique de la réduction en 5 α de la double liaison $\Delta_{4\text{-}5}$ (composés I à V et VII à XI) sans aucune réduction 5 β, et

— prééminence de l'oxydo-réduction 3 α.

L'apparition de ces métabolites de la progestérone constitue l'expression phénotypique normale du métabolisme de la progestérone chez les rats impubères (et également chez les rats femelles adultes).

En conclusion : la culture de cellules épithéliales de foie dans le milieu de culture selon l'invention offre un nouvel outil pour étudier l'expression et la régulation des fonctions hépatiques dans des cellules ne subissant pas les effets perturbants du sérum ou des hormones.

## Exemple 2

### Culture de cellules épithéliales de rein de singe en utilisant un dextrane en tant que biopolymère lipophile propagation de la lignée VERO

Indications générales :

La lignée VERO est une lignée cellulaire épithéliale de rein normal de singe, Cercopithecus aethiops, établie en mars 1962 par Y. Yasamura à l'Université de Chiba au Japon et déposée à l'American Type Culture Collection sous le n° ATCC CCL81. La souche cellulaire utilisée provient d'un flacon de VERO IM 153 obtenu le 24 avril 1983 au 153ème passage et est issue de l'échantillon provenant de l'ATCC.

Cette souche est divisée en deux sous-lignées, une sous-lignée est propagée normalement dans le milieu 199 [Morgan et Coll. Proc. Soc. Exp. Biol. Med. (1950), 73 1] additionné de 5 % de sérum de veau fœtal (SSM) et appelée lignée VERO 1, VERO 2... VERO 15 au fur et à mesure des passages dans le SSM et l'autre sous-lignée est propagée dans le milieu sans sérum (SFM) contenant de l'albumine et des acides gras, décrit à l'exemple 1, et ce dans des boîtes surfactées à l'aide de sérum selon la méthode exposée à l'exemple 1.

Au bout de trois passages de la sous-lignée croissant dans le SFM, une sous-lignée est initiée dans un nouveau milieu de culture sans sérum où le biopolymère lipophile de type albumine est remplacé par un biopolymère lipophile de type dextrane (Pharmacia, France) hautement purifié, sans métaux lourds, chimiquement et structurellement défini, au taux de 2 à 50 mg/l en maintenant la proportion des six acides gras utilisés selon l'invention, indiquée à l'exemple 1 mais avec une concentration totale finale de 0,152 μeq/l adsorbé sur le dextrane. Le milieu sans sérum et sans protéine vectrice ou SPFM (« Serum — and Protein — Free Medium ») est utilisé tel quel pour propager la lignée VERO S1, VERO S2 ... VERO S14 (S pour synthétique). La culture de la lignée VERO S dans le SFM contenant de l'albumine est arrêtée.

Le SPFM peut éventuellement être utilisé avec adjonction d'hormones et de facteurs de croissance. A titre indicatif on peut utiliser les hormones et facteurs de croissance mentionnés par G. Sato et Coll. dans Anal. Biochem. (1980) 102 : 235-270, aux doses indiquées dans cet article.

On peut par ailleurs remplacer le surfactage du substratum par le sérum en ajoutant au SPFM des fibronectines de différentes origines en tant que protéines d'attachement ainsi que d'autres protéines et polypeptides d'attachement tels que collagènes, polylysines.

Les différents polymères de chaînes isomériques de (α 1 → 3)-D-glucopyranosides réticulées par des groupes (α 1 → 6)-D-glucopyranosyle et qui constituent les dextranes homogènes tels que les dextranes de poids moléculaire 10 000, 40 000, 50 000, 70 000, 500 000 et 2 000 000 et les dextranes chargés tels que le sulfate de dextrane et le DEAE-dextrane, distingués par leur poids moléculaire moyen, les charges ou les groupements, peuvent être utilisés selon l'invention en tant que biopolymère lipophile. On préfère cependant le dextrane de poids moléculaire 500 000 et plus encore le dextrane de poids moléculaire 2 000 000 appelé dextrane T2000 (dénomination commerciale). Par commodité, le dextrane choisi, de préférence le dextrane T2000, est dissous en une solution mère 50 à 100 fois plus concentrée dans le milieu synthétique de base (BSM : « Basal Synthetic Medium ») afin de faciliter le stockage à + 4 °C après filtration stérilisante.

La propagation cellulaire est réalisée à chaque repiquage selon la méthode d'isolement et d'ensemencement des cellules décrite à l'exemple 1.

Le surfactage des boîtes est réalisé selon le mode indiqué à l'exemple 1 en utilisant le sérum disponible, par exemple de veau fœtal, de veau nouveau-né, de bovin adulte, de cheval ou humain.

Le milieu final est préparé selon les techniques classiques à partir d'un BSM. On choisit de préférence un BSM relativement riche tel que par exemple le milieu 199, le milieu MEMα (C.P. Stanners et Coll., Nature, New Biol. (1971) 230 : 310) avec ou sans la présence des 4 ribosides et des 4 désoxyribosides classiques (pentosides) et le milieu E de Williams (G.M. Williams et Coll., Exp. Cell Res. (1971) 69 : 105) ou tout autre BSM enrichi. A ce BSM sont ajoutés le ou les antibiotiques choisis, puis la solution mère stérile du dextrane T2000 pour aboutir à la concentration voulue, et pour finir, après avoir stérilisé le milieu par filtration, les solutions mères stériles d'acides gras en rendant parfaite la dissolution

par l'action des ultrasons pendant quelques minutes, puis en dernier lieu éventuellement les hormones et facteurs de croissance choisis en solution mère stérile.

On donne ci-après la composition du milieu 199 et du milieu E de Williams.

Milieu 199

| Composants | 199 avec sels de Earle mg/l | 199 avec sels de Hanks mg/l |
|---|---|---|
| L-alanine | 25,00 | 25,00 |
| L-arginine HCl | 70,00 | 70,00 |
| Acide L-aspartique | 30,00 | 30,00 |
| L-cystéine HCl | 0,099 | 0,099 |
| L-cystine | 23,66 | 23,66 |
| Acide L-glutamique | 66,82 | 66,82 |
| L-glutamine | 100,0 | 100,0 |
| Glutathion | 0,05 | 0,05 |
| Glycocolle | 50,00 | 50,00 |
| L-histidine HCl $H_2O$ | 21,88 | 21,88 |
| L-hydroxyproline | 10,00 | 10,00 |
| L-isoleucine | 20,00 | 20,00 |
| L-leucine | 60,00 | 60,00 |
| L-lysine HCl | 70,00 | 70,00 |
| L-méthionine | 15,00 | 15,00 |
| L-phénylalanine | 25,00 | 25,00 |
| L-proline | 40,00 | 40,00 |
| L-sérine | 25,00 | 25,00 |
| L-thréonine | 30,00 | 30,00 |
| L-tryptophane | 10,00 | 10,00 |
| L-tyrosine | 40,00 | 40,00 |
| L-valine | 25,00 | 25,00 |
| Acide L-ascorbique | 0,05 | 0,05 |
| Biotine | 0,01 | 0,01 |
| Calciférol | 0,10 | 0,10 |
| D-pantothénate de Ca | 0,01 | 0,01 |
| Chlorure de choline | 0,50 | 0,50 |
| Acide folique | 0,01 | 0,01 |
| 1-inositol | 0,05 | 0,05 |
| Ménadione | 0,019 | 0,019 |
| Acide nicotinique | 0,025 | 0,025 |
| Nicotinamide | 0,025 | 0,025 |
| Acide p-aminobenzoique | 0,05 | 0,05 |
| Pyridoxal HCl | 0,025 | 0,025 |

(Suite)

| Composants | 199 avec sels de Earle mg/l | 199 avec sels de Hanks mg/l |
|---|---|---|
| Pyridoxine HCl | 0,025 | 0,025 |
| Riboflavine | 0,01 | 0,01 |
| Thiamine HCl | 0,01 | 0,01 |
| α-tocophérol | 0,01 | 0,01 |
| Phosphate disodique | | |
| vitamine A | 0,115 | 0,115 |
| $CaCl_2$ $2H_2O$ | 264,9 | 185,5 |
| $Fe(NO_3)_3$ $9H_2O$ | 0,10 | 0,10 |
| KCl | 400,0 | 400,0 |
| $KH_2PO_4$ | | 60,00 |
| $MgSO_4$ $7H_2O$ | 200,0 | 200,0 |
| NaCl | 6800 | 8000 |
| $NaHCO_3$ | 2200 | 350,0 |
| $NaH_2PO_4$ $2H_2O$ | 158,3 | |
| $Na_2$ $HPO_4$ | | 47,50 |
| Adénine | 10,00 | 10,00 |
| 5'-AMP | 0,20 | 0,20 |
| ATP, sel de sodium | 10,00 | 10,00 |
| Cholestérol | 0,20 | 0,20 |
| 2-Déoxyribose | 0,50 | 0,50 |
| Glucose | 1000 | 1000 |
| Guanine HCl | 0,30 | 0,30 |
| Hypoxanthine | 0,30 | 0,30 |
| Ribose | 0,50 | 0,50 |
| Acétate de sodium | 36,71 | 36,71 |
| Rouge de phénol | 17,00 | 17,00 |
| Thymine | 0,30 | 0,30 |
| Tween 80 | 5,00 | 5.00 |
| Uracil | 0,30 | 0,30 |
| Xanthine | 0,30 | 0,30 |

Milieu de Williams E

| Composants | mg/l | Composants | mg/l |
|---|---|---|---|
| L-alanine | 90,00 | Chlorure de choline | 1,50 |
| L-arginine HCl | 60,50 | Acide folique | 1,00 |
| L-aspargine $H_2O$ | 20,00 | 1-inositol | 2,00 |
| Acide L-aspartique | 30,00 | Ménadione | 0,01 |

(Suite)

| Composants | mg/l | Composants | mg/l |
|---|---|---|---|
| L-cystéine HCl | 52,05 | Nicotinamide | 1,00 |
| L-cystine | 23,70 | Pyridoxal HCl | 1,00 |
| Acide L-glutamique | 50,00 | Riboflavine | 0,10 |
| L-glutamine | 292,0 | Thiamine HCl | 1,00 |
| Glutathion | 0,05 | DL-α-tocophérol (phosphate) | 0,01 |
| Glycine | 50,00 | Vitamine A | 0,10 |
| L-histidine HCl $H_2O$ | 20,30 | Vitamine B12 | 0,20 |
| L-isoleucine | 50,00 | $CaCl_2$ $2H_2O$ | 264,9 |
| L-leucine | 75,00 | $CuSO_4$ $5H_2O$ | 0,00009 |
| L-lysine HCl | 87,50 | $Fe(NO_3)_3$ $9H_2O$ | 0,0001 |
| L-méthionine | 15,00 | KCl | 400,0 |
| L-phénylalanine | 25,00 | $MgSO_4$ $7H_2O$ | 200,0 |
| L-proline | 30,00 | $MnCl_2$ $4H_2O$ | 0,0001 |
| L-sérine | 10,00 | NaCl | 6800 |
| L-thréonine | 40,00 | $NaHCO_3$ | 2200 |
| L-tryptophane | 10,00 | $NaH_2PO_4$ $2H_2O$ | 158,3 |
| L-tyrosine | 35,00 | $ZnSO_4$ $7H_2O$ | 0,0002 |
| L-valine | 50,00 | Glucose | 2000 |
| Acide L-ascorbique | 2,00 | Linoléate de méthyle | 0,03 |
| Biotine | 0,50 | Rouge de phénol | 10,00 |
| Calciférol | 1,00 | Pyruvate de sodium | 25,00 |
| D-pantothénate de Ca | 1,00 | | |

Résultats :

La lignée VERO, sous-lignée VERO S, est entretenue pendant seize passages dans le SPFM de base (BSPFM) préparé comme suit à partir de :

— un milieu synthétique chimiquement défini, le BSM E de Williams, qui est préféré parce qu'il contient toutes les vitamines dont la vitamine A et la vitamine C (il peut être remplacé par tout autre milieu de même type le 199 ou le MEMα, avec ou sans pentosides), complété avec les taux requis de L-glutamine selon la forme de présentation, milieu en poudre ou milieu liquide et/ou de bicarbonate selon le pourcentage de $CO_2$, 5 % ou 10 %, mélangé à l'air atmosphérique de l'étuve à culture ;

— de l'eau ultrapure répondant aux normes ASTM-1 [recueil ASTM D-1193-70 (1970)] ou mieux de l'eau purifiée selon la méthode de Chessebeuf et Coll. [C. R. Soc. Biol. Paris (1979), 173, 469-582] et

— un ou des antibiotiques à large spectre, de préférence la gentamycine au taux de 50 mg/l.

A ce niveau de préparation le milieu est filtré stérilement et conservé à 4 °C pendant une période n'excédant pas une dizaine de jours à cause de la dégradation de la L-glutamine.

Au moment de l'utilisation, les composés suivants sont ajoutés extemporanément :

— solution mère stérile de dextrane T2000 conservée à + 4 °C pour obtenir la concentration préférée de 10 mg/l ;

— solution mère éthanolique stérile d'acides gras conservée à 4 °C pour obtenir la concentration préférée finale de 152 nanoéquivalents (nEq)/litre contenant les six acides gras libres utilisés selon l'invention, dans les proportions indiquées à l'exemple 1.

Première série d'essais : effet de l'adjonction d'hormones ou de facteurs de croissance.

Dans le BSPFM ainsi préparé, utilisé seul ou avec adjonction d'hormones ou de facteurs de croissance, on cultive la lignée VERO au 15$^{eme}$ passage en réalisant le surfactage par le sérum de veau fœtal (SVF) et on étudie la croissance (nombre de cellules) en fonction du temps.

Composition des milieux de culture :
— milieu 1 : BSPFM seul
— milieu 2 : BSPFM + insuline : 1,25 mg/l ;
— milieu 3 : BSPFM + transferrine-fer (saturée ou demi-saturée en fer) : 10 mg/l ;
— milieu 4 : BSPFM + « epidermal growth factor » EGF : 12,5 μg/l ;
— milieu 5 : BSPFM + insuline : 1,25 mg/l + transferrine-fer : 10 mg/l + EGF : 12,5 μg/l.

Les cinq milieux assurent une prolifération à partir d'un inoculum de 500 000 à 700 000 cellules (de viabilité 98 à 100 %) par boîte de 20 $cm^2$, aboutissant à un taux de confluence cellulaire compris entre $4 \cdot 10^6$ et $5,25 \cdot 10^6$ cellules/20 $cm^2$ au sixième jour après l'ensemencement. Au huitième jour après l'ensemencement les meilleurs résultats sont obtenus avec les milieux 1 et 3. Le taux de cellules est de $4,9 \cdot 10^6$ cellules/20 $cm^2$ pour le milieu 1, et de $5,7 \cdot 10^6$ cellules/20 $cm^2$ pour le milieu 3. Le taux de multiplication cellulaire dans le milieu 1 est de 9,8 et le temps de doublement de la population cellulaire est de 19 h 35 min. Pour le milieu 3, le taux de multiplication cellulaire est de 7,2 et le temps de doublement de la population cellulaire de 25 h 15 min.

La figure 1 est une représentation graphique des résultats obtenus dans cet essai. Elle comprend une ordonnée en logarithmes népériens, $\log_n$ N/No, où N est le nombre de cellules au temps t et No le nombre de cellules au temps t = 0 correspondant au comptage effectué 12 h après l'inoculation. Les numéros des courbes correspondent aux numéros des milieux concernés. Les cinq inoculums prolifèrent d'une façon identique. On note cependant une certaine supériorité du milieu 1. L'une des cultures (milieu 4) a été arrêtée au 3ème jour.

Toujours dans le cadre de cette première série d'essais, on étudie l'influence d'autres hormones et facteurs de croissance sur la croissance de la lignée VERO S15 dans des boîtes de culture surfactées au SVF.

Les milieux utilisés sont :
— milieu 1 : sans hormone ni facteur de croissance (comme précédemment) ;
taux de multiplication cellulaire : 8,8 ;
temps de doublement de la population cellulaire : 21 h 49 min ;
— milieu 4 : (comme précédemment) avec EGF : 12,8 μg/ml :
taux de multiplication cellulaire : 9,8 ;
temps de doublement de la population cellulaire : 19 h 35 min ;
— milieu 5 : BSPFM du milieu 1 + MSA (facteur d'activité stimulatrice de la multiplication : « Multiplication Stimulating Activity ») : 1 μg/l ;
taux de multiplication cellulaire : 6,5 ;
temps de doublement de la population cellulaire : 29 h 32 min ;
— milieu 6 : BSPFM du milieu 1 + dexaméthasone : 50 μg/l ;
taux de multiplication cellulaire : 6,5 ;
temps de doublement de la population cellulaire : 29 h 32 min ;
— milieu 7 : BSPFM du milieu 1 + putrescine : 100 μmoles/l ;
taux de multiplication cellulaire : 8,2 ;
temps de doublement de la population cellulaire : 23 h 25 min ;

On constate que seul le facteur de croissance EGF augmente légèrement le taux de multiplication cellulaire tandis qu'une autre hormone, la dexaméthasone, et le facteur de croissance cellulaire MSA n'apportent pas de grande amélioration par rapport au milieu BSPFM. La putrescine apporte une stimulation mais provoque ensuite une réaction toxique au moment de la confluence. Utilisée dans une autre expérience à un taux 10 fois plus faible, 10 μmoles/l, la putrescine n'apporte pas de différence par rapport au milieu témoin BSPFM.

La figure 2 donne une représentation graphique, du même type que pour la figure 1, des résultats obtenus avec les milieux 1, 4, 5, 6 et 7 ci-dessus.

Cette représentation montre bien les effets négatifs du MSA et de la putrescine, l'absence d'effet de la dexaméthasone et l'effet légèrement stimulateur de l'EGF par rapport à l'effet du BSPM sur la croissance cellulaire. Pour une production industrielle de la lignée VERO S, l'addition d'EGF au BSPFM composé du BSM, le milieu E de Williams, gentamycine : 50 mg/l + dextrane T2000 : 10 mg/l + 6 acides gras utilisés selon l'invention : 152 nEq/l, n'apporte pas d'amélioration économiquement intéressante.

Deuxième série d'essais : culture sur SSM (essais comparatifs) :

On étudie, au 16ème passage, la croissance de la lignée VERO 15 poussant dans le SSM contenant 5 % de sérum depuis le 1er passage issu de la lignée VERO IM 153, après division en deux sous-lignées repiquées dans deux BSM différents en présence de 5 % de sérum :
— milieu 1 : BSM Williams E + gentamycine : 50 mg/l + 5 % de sérum veau fœtal (SVF) ;
— milieu 2 : BSM constitué de 50 % de 199 et de 50 % de MEMα sans pentosides + gentamycine : 50 mg/l + 5 % de SVF.

La confluence est atteinte dans les deux cas en 6 jours avec un taux moyen de $4,1 \cdot 10^6$ cellules/20 $cm^2$ pour un inoculum initial de $0,5 \cdot 10^6$ cellules. Le taux de multiplication cellulaire est donc de 8,5 en 6 jours, et le temps de doublement de la population cellulaire est de 17 h.

On ne constate aucune différence entre les deux types de BSM lorsqu'ils sont complétés par du sérum de type SVF.

Troisième série d'essais : influence du BSM dans le cas d'une culture sur SPFM.

On étudie la croissance cellulaire de la lignée VERO S17, 17ème passage dans les deux SPFM correspondants aux deux BSM de la deuxième série d'essais, à savoir le milieu E de Williams d'une part et le mélange de 50 % de milieu 199 et de 50 % de milieu MEMα sans pentosides, en absence de sérum et de protéines, pour deux cultures menées parallèlement dans des boîtes de culture de 20 $cm^2$ de surface surfactées soit par du SVF, soit en remplaçant le surfactage au sérum par adjonction de fibronectine humaine (FH) au milieu. Ces milieux sont :

— milieu 1 : BSM E de Williams + gentamycine : 50 mg/l + dextrane T2000 : 10 mg/l + 6 acides gras : 152 nEq/l ; surfactage du substratum au SVF ;

— milieu 2 : identique au milieu 1 mais avec addition au milieu de FH : 9 µg/20 $cm^2$, soit 2 µg/ml de SPFM (sans surfactage par du sérum) ;

— milieu 3 : BSM composé de 50 % de 199 et 50 % de MEMα sans pentosides + gentamycine : 50 mg/l + dextrane T2000 : 10 mg/l + 6 acides gras : 152 nEq/l ; surfactage par le SVF ;

— milieu 4 : identique au milieu 3, mais avec addition de FH : 2 µg/ml (sans surfactage par du sérum).

Partant d'un inoculum de 363 000 cellules, la culture de la lignée VERO S17 réalisée avec le milieu SPFM composé du BSM E de Williams (milieux 1 et 2) montre une prolifération meilleure que la lignée VERO 15 en présence de sérum (deuxième série d'essais) puisqu'au bout de 6 jours le nombre de cellules se situe à une moyenne de $4{,}9 \cdot 10^6$ cellules/20 $cm^2$, correspondant à un taux de multiplication cellulaire de 13,5 et le temps de doublement de la population cellulaire est de 14 h 13 min pour la lignée VERO S17 contre les valeurs respectives de 8,5 et de 17 h pour lignée VERO 15 croissant dans le même BSM E de Williams additionné de 5 % de sérum.

Les deux cultures homologues réalisées dans le BSM constitué d'un mélange en proportions égales de milieu 199 et de milieu MEMα atteignent le plateau de croissance au bout de 4 jours pour un nombre de cellules de la lignée VERO S17 allant de $2{,}25 \cdot 10^6$ à $2{,}35 \cdot 10^6$ cellules/20 $cm^2$. Le taux de multiplication cellulaire est de 3,3 et le temps de doublement de la population cellulaire de 29 h.

Cet exemple montre l'importance du choix du BSM dans le cas de la culture sans sérum.

La figure 3 réunit sur un même graphique les résultats des deuxième et troisième séries d'essais représentés de façon semi-logarithme où l'ordonnée est le logarithme népérien, $\log_n$ N/No, pour N = nombre de cellules au temps t et No = nombre de cellules au temps t0. Ce t0 correspond en fait au comptage 6 h après l'ensemencement.

Cette figure permet de comparer d'une part le type de BSM (milieu E de Williams ou 50 % de milieu 199 + 50 % de milieu MEMα sans pentosides), entrant dans la composition du SSM à 5 % de sérum de veau fœtal ou du SPFM, et d'autre part la méthode d'ancrage des cellules des cultures SPFM, soit par surfactage au SVF incubé seul 12 h dans la boîte puis rejeté avec rinçage subséquent, soit par addition au SPFM de FH au taux de 2 mg/l sans aucune autre préparation du substratum avant le repiquage. Les numéros des courbes correspondent aux numéros des milieux de culture de composition :

— milieu 1 : BSM : E de Williams + gentamycine : 50 mg/l + 5 % de SVF ;

— milieu 2 : BSM : 50 % de 199 + 50 % de MEMα sans pentosides + gentamycine : 50 mg/l + 5 % de SVF ;

— milieu 3 : BSM : E de Williams + gentamycine : 50 mg/l + dextrane T2000 : 10 mg/l + 6 acides gras : 152 nEq/l ; surfactage par le SVF ;

— milieu 4 : BSM : E de Williams + gentamycine : 50 mg/l + dextrane T2000 : 10 mg/l + 6 acides gras : 152 nEq/l + FH : 2 mg/l ; sans aucun autre surfactage ;

— milieu 5 : BSM : 50 % de 199 + 50 % de MEMα sans pentosides + gentamycine : 50 mg/l + dextrane T2000 : 10 mg/l + 6 acides gras : 152 nEq/l ; surfactage par le SVF ;

— milieu 6 : BSM : 50 % de 199 + 50 % de MEMα sans pentosides + gentamycine : 50 mg/l + dextrane T2000 : 10 mg/l + 6 acides gras : 152 nEq/l + FH : 2 mg/l, sans aucun autre surfactage.

La figure 3 démontre la déficience du mélange BSM constitué de 50 % de milieu 199 + 50 % de milieu MEMα sans pentosides. Cette déficience n'est pas compensée par la composition du SPFM. Le problème de l'ancrage cellulaire sur le substratum n'intervient pas puisque dans le cas des quatres cultures SPFM, le surfactage par le SVF ou l'addition de FH au milieu sans autre surfactage conduit à des croissances cellulaires comparables dans un même groupe de BSM (milieux 3 et 4, ou milieux 5 et 6). Dans le cas du SPFM constitué de BSM E de Williams, les croissances cellulaires (milieux 3 et 4) restent proches de celles des cultures en présence de sérum (milieux 1 et 2).

Quatrième série d'essais : effet de la taille de l'inoculum.

On a effectué trois comparaisons avec les lignées VERO S15, VERO S17 et VERO 15 :

i) entre deux taux de cellules à l'ensemencement, soit 23 000 cellules/$cm^2$, soit 42 500 cellules/$cm^2$ dans des boîtes de culture de 20 $cm^2$,

ii) entre le BSM E de Williams contenant du sérum pour la lignée VERO croissant dans du sérum depuis 1962 et au 15ème passage au laboratoire (VERO 15) et le milieu SPFM à partir du même BSM E de Williams,

iii) entre deux passages de la lignée VERO poussant dans le SPFM depuis soit 15 passages : VERO S15, ou 17 passages : VERO S17.

Description des essais et résultats :

Milieu 1 : BSM E de Williams + gentamycine 50 mg/l + dextrane T2000 : 10 mg/l + 6 acides gras : 152 nEq/l ; surfactage par le SVF ; passage VERO S17 ;
Inoculum 42 500 cellules/cm$^2$, soit 850 000 cellules/20 cm$^2$ ;
taux de multiplication cellulaire ; 5,7 · 10$^6$/850 000 = 6,7 ;
temps de doublement de la population cellulaire : 192/6,7 = 28 h 40 min.

Milieu 2 : même BSM que précédemment ; surfactage par le SVF ; passage VERO S15 :
Inoculum 23 500 cellules/cm$^2$, soit 470 000 cellules/20 cm$^2$ ;
taux de multiplication cellulaire : 4,95 · 10$^6$/470 000 = 10,5 ;
temps de doublement de la population cellulaire : 192/10,5 = 18 h 17 min.

Milieu 3 : même BSM que précédemment ; surfactage par le SVF ; passage VERO S15 :
Inoculum 42 500 cellules/cm$^2$, soit 850 000 cellules/20 cm$^2$ ;
taux de multiplication cellulaire : 5,25 · 10$^6$/850 000 = 6,6 ;
temps de doublement de la population cellulaire : 192/6,6 = 29 h 4 min.

Milieu 4 : même BSM que précédemment ; surfactage FH ; passage VERO S17 ;
Inoculum 23 700 cellules/cm$^2$, soit 474 000 cellules/20 cm$^2$ ;
taux de multiplication cellulaire : 4,9 · 10$^6$/474 000 = 10,4 ;
temps de doublement de la population cellulaire : 192/10,4 = 18 h 34 min.

Milieu 5 : même BSM que précédemment + gentamycine 50 mg/l + 5 % de SVF ; passage VERO 15 ;
Inoculum 23 500 cellules/cm$^2$, soit 470 000 cellules/20 cm$^2$ ;
taux de multiplication cellulaire : 4,9 · 10$^6$/470 000 = 10,4 ;
temps de doublement de la population cellulaire : 192/10,4 = 18 h 28 min.

L'effet de la taille de l'inoculum est le même, que ce soit dans la culture en présence de SVF ou dans les cultures SPFM ; milieux 1 et 3 ou milieux 2 et 4.

Dans le cas d'un inoculum de 23 500 cellules/cm$^2$, malgré la présence de sérum (milieu 5), la croissance cellulaire n'est pas plus active que dans le cas du milieu SPFM correspondant (milieu 2) et ne rattrape pas, en nombre de cellules, la croissance d'un inoculum de 42 500 cellules/cm$^2$ dans les milieux SPFM (milieux 1 et 3).

Cette étude permet de constater que pour les taux d'inoculation de l'ordre de 500 000 cellules/20 cm$^2$, le SPFM est caractérisé par les paramètres de culture suivants : taux de multiplication cellulaire de 10,5 et temps de doublement de la population cellulaire de 18 h 17 min, similaires à ceux obtenus dans le SSM à 5 % de sérum, respectivement 10,4 et 18 h 28 min. Au taux d'inoculation plus élevé de 850 000 cellules/20 cm$^2$, les paramètres dans le cas de deux cultures SPFM parallèlement sont semblables.

## Exemple 3

### Croissance de la lignée de myélome SP2/O-Ag-14

La lignée de myélome de souris SP2/O-Ag-14 [M. Shulman et Coll., Nature (1978), 276, 269-270] est très utilisée pour obtenir des hybridomes de cellules myélomateuses et de lymphocytes d'animaux immunisés contre un ou plusieurs antigènes afin d'obtenir une ou des lignées d'hybridomes sécrétant chacune un anticorps monoclonal.

La croissance de la lignée SP2/O-Ag-14 et des hybridomes sécrétant des anticorps monoclonaux qui en sont issus est réalisée actuellement dans un milieu contaminant 10 % de sérum. L'addition de sérum est responsable de la présence d'anticorps hétérospécifiques appartenant à l'espèce animale sur laquelle il a été prélevé. Ces anticorps étrangers contaminent l'anticorps monoclonal sécrété par l'hybridome. En effet, le taux de sécrétion d'un anticorps monoclonal d'un hybridome se situe le plus souvent entre 5 et 100 mg d'anticorps spécifique par litre de milieu. Selon le taux des immunoglobulines étrangères apportées par le sérum ajouté à raison de 10 % au milieu de culture, la contamination se situe entre 700 et 1 400 mg/l de ces anticorps étrangers. Il est cependant possible de faire pousser des cellules d'hybridome dans un milieu sans sérum et souvent en présence d'albumine afin d'obtenir un milieu de culture dont l'immunoglobuline majoritaire soit l'anticorps spécifique monoclonal, par rapport à d'autres protéines sécrétées par l'hybridome, aux protéines provenant de la lyse cellulaire, et à des protéines pouvant être ajoutées au milieu de culture en tant que facteurs vecteurs, hormonaux, de liaison ou de croissance.

On étudie la croissance de la lignée de myélome de souris, SP2/O-Ag-14 dans les milieux de culture SPFM.

La croissance de la lignée de myélome SP2/O-Ag-14 est effectuée dans des flacons de culture de 25 $cm^2$ de surface. La pénicilline, 50 U/ml, et la streptomycine, 50 μg/ml habituellement utilisées, sont utilisées ici également pour ne pas apporter de facteur de modification supplémentaire à la méthode de culture. Les cultures sont réalisées en absence de surfactage par le SVF mais en ajoutant de la FH au SPFM. Le SPFM est composé de BSM MEMα sans pentosides.

Les milieux utilisés ont les compositions suivantes (les comptages cellulaires sont donnés en $10^6$ cellules) :

— Milieu 1 : milieu standard de culture de cellules de myélome : RPMI 1640 + glucose à 5,6 g/l, + pyruvate de Na : 1 mmole/l + 10 % de SVF.
Taux de multiplication cellulaire (TMC) : 6,3/1,05 = 6,0 ;
temps de doublement de la population cellulaire (TDPC) : 83/6,0 = 13 h 30 min.

— Milieu 2 : même milieu que le précédent où le RPMI est remplacé par le MEMα sans pentosides.
TMC : 6,3/1,17 = 5,4 ; TDPC : 83/5,4 = 15 h 25 min.

— Milieu 3 : BSM MEMα sans pentosides + glucose à 5,6 g/l + dextrane T2000 : 10 mg/l + 6 acides gras : 152 nEq/l, + insuline : 2 mg/l + transferrine-fer : 2 mg/l + EGF : 20 μg/l.
TMC : 3,88/0,95 = 4,1 ; TDPC : 83/4,1 = 20 h 19 min.

— Milieu 4 : même SPFM complet que celui du milieu 3 + FH : 10 mg/l.
TMC : 5,16/1,06 = 4,9 ; TDPC : 83/4,9 = 17 h 3 min.

— Milieu 5 : même SPFM complet que celui du milieu 3 + (« endothelial cell growth supplement » ECGS) : 60 mg/l.
TMC : 5,59/1,25 = 4,5 ; TDPC : 83/4,5 = 18 h 34 min.

— Milieu 6 : même SPFM que le milieu 5 + FH : 10 mg/l.
TMC : 5,9/1,0 = 5,9 ; TDPC : 83/5,9 = 14 h 10 min.

Le nombre de cellules comptées tient compte des cellules qui se sont ancrées sur le substratum et des cellules en suspension dans le milieu. L'ancrage des cellules sur le substratum est plus rapide, et la prolifération est initiée plus tôt, dans les milieux 1 et 2 contenant du sérum.

Le milieu 3 montre que sans FH et sans ECGS, la croissance apparaît plus rapide au départ, analogue à celle des milieux 1 et 2. En fait peu de cellules sont ancrées sur le substratum, les cellules se divisent surtout en suspension sans passer par la phase d'attachement et meurent. Les milieux 4 et 5 assurent une croissance similaire se rapprochant de celle dans le SSM grâce à la présence de la FH (milieu 4) ou de l'ECGS seuls (milieu 5 un peu plus actif que le milieu 4). Ils améliorent la stimulation de la croissance par rapport au milieu 3, mais surtout ramènent la mortalité cellulaire à celle des milieux 1 et 2 en permettant un très bon ancrage des cellules sur le substratum. Lorsque les deux facteurs FH et ECGS sont ajoutés ensemble dans le milieu 6, la croissance cellulaire est encore plus efficace et se rapproche de celle des milieux 1 et 2 contenant du sérum. Il faut rappeler que le substratum n'a subi dans le cas des SPFM (milieux 3 à 6) aucun surfactage physicochimique par le sérum ou des polymères naturels ou artificiels d'ancrage. Donc en absence de sérum pour l'ancrage de cellules, il se produit dans le cas des milieux 4, 5 et 6 un temps de latence correspondant à la période d'ancrage assuré par les cellules elles-mêmes sur le substratum. Néanmoins, la reprise de la prolifération cellulaire se fait au même temps, à 35 h, comme dans le cas des milieux 1 et 2 contenant du sérum, s'accélérant fortement dans les milieux SPFM 4, 5 et 6 à partir de la $75^{ème}$ heure, pour aboutir à la $83^{ème}$ heure à des taux de population cellulaire dans ces cultures voisins des taux dans les milieux 1 et 2 en présence de sérum.

La confluence atteinte pour les différentes cultures en présence de sérum (milieux 1 et 2) ou dans les milieux SPFM 4 et surtout 5 et 6 correspond à un nombre de cellules entre 5 et $7 \cdot 10^6$ cellules/20 $cm^2$ dans 5 ml de milieu.

## Exemple 4

### Croissance d'un hybridome obtenu à partir de lymphocytes d'animaux immunisés contre un antigène viral de rotavirus

Les rotavirus sont responsables de maladies diarrhéiques dépendantes ou indépendantes des maladies de la malnutrition chez l'homme comme chez certaines espèces animales. La mise à la disposition du médecin d'un diagnostic étiologique est un atout important. Il peut être fait d'une façon efficace à l'aide d'anticorps spécifiques des différentes souches virales.

L'exemple donné correspond à la croissance d'un hybridome SP2/O-Ag-14 du rotavirus Rota IV-21.

Le milieu de culture habituel en présence de sérum est le BSM RPMI 1640 + glucose à 5,6 g/l + pyruvate de Na : 1 mmole/l. Ce milieu peut être remplacé par le BSM E de Williams. Dans cet exemple, la croissance de cet hybridome a été réalisée dans ce dernier BSM + glucose à 5,6 g/l + gentamycine : 50 mg/l + 10 % SVF.

Le milieu de culture SPFM, sans sérum et sans protéine vectrice est composé, soit de BSM RPMI 1640, soit de BSM E de Williams, chacun contenant 5,6 g/l de glucose + pyruvate de Na : 1 mmole/l + dextrane T2000 : 10 mg/l + 6 acides gras : 7,6 μEq/l (voir Exemple 1) + gentamycine : 50 mg/l.

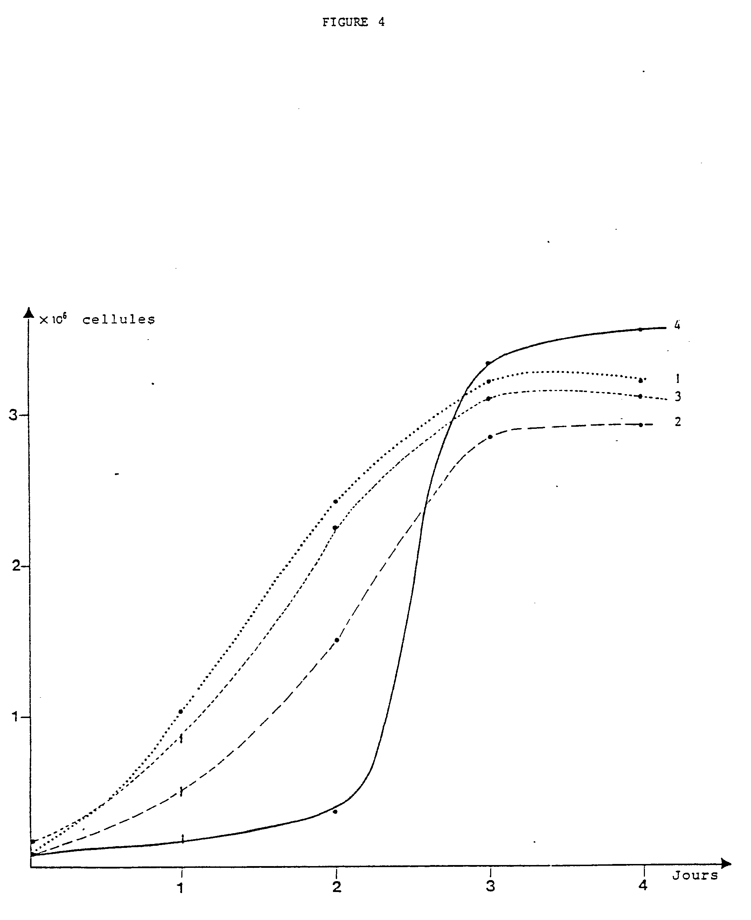

La figure 4 montre les courbes de croissance cellulaire de l'hybridome Rota IV-21 dans différents milieux et différentes conditions (les comptages cellulaires sont donnés en $10^6$ cellules) :

— Courbe 1 : croissance de l'hybridome Rota IV-21 dans du BSM E de Williams complété par du glucose à 5,6 g/l + pyruvate de Na : 1 mmole/l + gentamycine : 50 mg/l + 10 % de SVF (milieu 1) ; taux d'inoculation : 100 000 cellules/20 $cm^2$.

TMC : 3,23/0,1 = 32,3 ; TDPC : 72/32,3 = 2 h 14 min.

— Courbe 2 : elle représente le cas d'une sous-lignée de Rota IV-21 ayant auparavant déjà proliféré pendant trois passages dans le milieu SPFM utilisé ci-dessous et qui comprend le BSM E de Williams – gentamycine : 50 mg/l + glucose à 5,6 g/l + dextrane T2000 : 10 mg/l + acides gras : 7,6 μEq – transferrine-fer : 5 mg/l + insuline : 1,25 mg/l + EGF : 5 μg/l (milieu 2) ; inoculum : 100 000 cellules/20 $cm^2$.

TMC : 2,85/0,1 = 28,5 ; TDPC : 72/28,5 = 2 h 32 min.

— Courbe 3 : même SPFM complet que le milieu 2, FH : 2 mg/l (milieu 3) pour la même sous-lignée adaptée depuis trois passages à la croissance dans le milieu 2 ; inoculum : 150 000 cellules/20 $cm^2$.

TMC : 3,21/0,15 = 21,4 ; TDPC : 72/21,4 = 3 h 22 min.

— Courbe 4 : même SPFM complet que dans le milieu 3 mais appliqué à une sous-lignée proliférant au passage précédent dans le milieu 1 contenant du sérum ; inoculum : 100 000 cellules/20 $cm^2$.

TMC : 3,34/0,1 = 33,4 ; TDPC : 72/33,4 = 2 h 9 min.

On observe en microscopie à fluorescence la réaction des anticorps anti-rotavirus sécrétés par les hybridomes Rota IV-21 croissant dans les milieux de culture SSM et SPFM de la lignée SSM et des sous-lignées SPFM, contre des cellules infectées par le Rotavirus. On observe parallèlement les témoins correspondants où les cellules infectées sont incubées dans les milieux de culture frais respectifs.

Les témoins ne montrent aucune réaction histochimique immunofluorescente sur les cellules.

On constate par ailleurs que la production d'anticorps est très semblable en comparant les trois milieux de culture sans sérum et sans protéines vectrices, SPFM, 2, 3 et 4 par rapport au SSM contenant 10 % de sérum.

On en déduit que la production d'anticorps s'effectue parallèlement au taux de prolifération des hybridomes dans ces milieux de culture et proportionnellement au taux de cellules à la confluence en fin de croissance de la culture (phase en plateau au début du 3ème jour). Ramenée au taux de cellules, elle est identique dans tous les milieux, que ce soit dans le milieu de culture contenant du sérum ou dans le milieu de culture sans sérum et sans protéine vectrice.

Grâce à l'invention il est maintenant possible non seulement d'entretenir une population cellulaire d'hybridomes dans un milieu sans sérum mais de la faire proliférer dans un milieu ne contenant ni sérum, ni protéine vectrice. Cette prolifération dans ce milieu ne se produit que parce qu'il conserve aux hybridomes leur capacité d'ancrage sur le substratum. Il n'est donc pas nécessaire, comme dans le cas de la technique de culture proposée par Cleveland et Coll. (J. Immunol. Methods (1983), 56, 221-234), de procéder dans un premier temps à l'ancrage des cellules hybridomes sur le substratum à l'aide d'un milieu de culture contenant du sérum et de les faire proliférer pour aboutir à une confluence de cellules attachées en équilibre avec les cellules en suspension. Dans le procédé de Cleveland et Coll., à la confluence, le milieu contenant du sérum peut être remplacé par le milieu d'entretien sans sérum des cellules afin de faire produire des anticorps par ces cellules dans le milieu de culture sans immunoglobulines hétérospécifiques. Néanmoins, ce milieu ne permet en aucune façon d'accroître par prolifération la biomasse de la souche cellulaire. Donc les cellules, dont la production d'anticorps peut être limitée à une durée de vie limitée de la lignée, sont perdues autant pour la propagation de la souche que pour l'accroissement de la production de l'anticorps monoclonal.

Le milieu de culture sans sérum selon l'invention permet l'entretien et la prolifération de la lignée de l'hybridome qui doit passer par l'intermédiaire de l'ancrage cellulaire et de la prolifération cellulaire pour aboutir à une production de l'anticorps monoclonal rentable. La rentabilité est fortement accrue par les économies réalisées sur la purification de l'anticorps monoclonal à partir du milieu de culture ne contenant pas d'anticorps étrangers.

L'exemple de croissance cellulaire d'hybridome dans les milieux 2 et 3 ci-dessus a été réalisé sur une sous-lignée d'hybridome qui avait déjà subi trois passages dans le milieu 2. Ceci démontre que les effets rémanents du sérum ne peuvent pas être invoqués pour expliquer l'ancrage et ensuite la prolifération des cellules d'hybridome dans le milieu sans sérum. En effet, l'adaptation de la croissance d'une culture faite auparavant dans le milieu SSM (milieu 1) au milieu SPFM (milieu 4) démontre l'existence d'un temps de latence pour assurer l'ancrage cellulaire pendant 48 h avant le démarrage de la prolifération cellulaire dans le milieu SPFM. Dans le cas des cultures SPFM des milieux 2 et 3, l'ancrage et la prolifération cellulaire progressent simultanément.

**Revendications**

1. Milieu de culture de cellules animales, sans sérum, caractérisé en ce qu'il est constitué :

a) d'un milieu synthétique de base conçu pour la culture de cellules animales ;

b) de 0,05 à 30 μeq/l d'un mélange des six acides gras : acide palmitique, acide cis-palmitoléique, acide stéarique, acide cis-oléique, acide cis-linoléique et acide cis-linolénique présents chacun sous la forme de l'acide libre ou de l'un de ses esters, dans les proportions suivantes, rapportées à l'acide gras libre :

— de 0,0155 à 24,0 µmole/l d'acide palmitique ;
— de 0,0058 à 9,0 µmole/l d'acide cis-palmitoléique ;
— de 0,0014 à 2,0 µmole/l d'acide stéarique ;
— de 0,0067 à 10,0 µmole/l d'acide cis-oléique ;
— de 0,0178 à 27,0 µmole/l d'acide cis-linoléique ; et
— de 0,0028 à 4,0 µmole/l d'acide cis-linolénique

adsorbés sur

c) au moins un biopolymère lipophile présent en une quantité capable d'assurer la dissolution des acides gras ou de leurs esters présents dans le milieu.

2. Milieu de culture selon la revendication 1, caractérisé en ce que le biopolymère lipophile est constitué par de l'albumine délipidée ou non, de préférence en une quantité de 2 à 6 g/l de milieu de culture ou par un dextrane, de préférence en une quantité de 0,5 à 200 mg/l de milieu de culture.

3. Milieu de culture de cellules animales, sans sérum, caractérisé en ce qu'il est constitué :

a) d'un milieu synthétique de base conçu pour la culture de cellules animales ;

b) de 0,05 à 30 µeq/l d'un mélange des six acides gras : acide palmitique, acide cis-palmitoléique, acide stéarique, acide cis-oléique, acide cis-linoléique et acide cis-linolénique présents chacun sous la forme de l'acide libre ou de l'un de ses esters, dans les proportions suivantes, rapportées à l'acide gras libre :

— de 0,0155 à 24,0 µmole/l d'acide palmitique ;
— de 0,0058 à 9,0 µmole/l d'acide cis-palmitoléique ;
— de 0,0014 à 2,0 µmole/l d'acide stéarique ;
— de 0,0067 à 10,0 µmole/l d'acide cis-oléique ;
— de 0,0178 à 27,0 µmole/l d'acide cis-linoléique ; et
— de 0,0028 à 4,0 µmole/l d'acide cis-linolénique

adsorbés sur

c) au moins un dextrane présent en une quantité capable d'assurer la dissolution des acides gras ou de leurs esters présents dans le milieu ; et éventuellement d'hormones et/ou de facteurs de croissance.

4. Milieu de culture selon la revendication 3, caractérisé en ce qu'il contient de 0,5 à 200 mg/l de dextrane.

5. Milieu de culture selon la revendication 3 ou 4, caractérisé en ce que le dextrane utilisé est le dextrane homogène de poids moléculaire 2 000 000.

6. Milieu de culture selon l'une des revendications 1 à 5, caractérisé en ce qu'il comprend en outre une concentration non cytotoxique, pour toute la durée de passage de la culture, d'un antibiotique, de préférence à large spectre.

7. Milieu de culture selon la revendication 6, caractérisé en ce qu'il comprend en tant qu'antibiotique, de la gentamycine à raison de 25 à 100 mg/l.

8. Milieu de culture selon l'une des revendications à 7, caractérisé en ce que les six acides gras y sont présents à l'état d'acides libres.

9. Milieu de culture selon l'une des revendications 1 à 8, caractérisé en ce que le milieu synthétique de base est constitué par un milieu choisi parmi :

— les milieux de Ham,
— le milieu Waymouth MB 752/1,
— les milieux RPMI 1629 et 1640,
— le milieu de Eagle,
— les milieux de Eagle modifiés,
— le milieu E de Williams,
— le milieu 199 et les milieux dérivés de type MEM et MEMα.

10. Procédé pour la culture primaire de cellules animales utilisant le milieu de culture selon l'une des revendications 1 à 9, caractérisé en ce qu'il consiste essentiellement à :

1) prélever le tissu renfermant les cellules à cultiver et le découper très finement, dans un milieu de culture de base, de préférence dans le milieu de base devant être utilisé mais ne contenant pas d'ions $Ca^{2+}$ et $Mg^{2+}$, tiédi (30-37 °C), dans des conditions aseptiques ;

2) après rinçage, de préférence dans le milieu de base devant être utilisé mais ne contenant pas d'ions $Ca^{2+}$ et $Mg^{2+}$, soumettre le tissu à digestion enzymatique,

3) transférer la suspension de cellules ainsi libérées dans un milieu de base, de préférence celui utilisé pour la culture, centrifuger en poursuivant parallèlement la digestion du tissu restant,

4) mettre le culot de centrifugation en suspension dans le milieu de culture contenant éventuellement un agent de surfactage du substratum de culture puis,

5) inoculer la suspension ainsi obtenue sur le substratum de culture préalablement ou simultanément surfacté,

6) incuber dans un incubateur étanche, saturé par la vapeur d'eau, en présence d'un mélange d'azote, d'oxygène et de gaz carbonique dans des proportions utilisables en biologie de la respiration cellulaire, en renouvelant périodiquement le milieu de culture,

les étapes 3) à 6) étant répétées sur les différentes fractions de cellules désirées dissociées, obtenues

successivement à l'étape 2), en utilisant à chaque fois un nouveau substratum de culture.

11. Procédé de culture primaire selon la revendication 10, caractérisé en ce que la digestion enzymatique est effectuée à l'aide de collagénase ou de trypsine, de préférence de trypsine.

12. Procédé de culture primaire selon la revendication 10 ou 11, caractérisé en ce que le surfactage du substratum est effectué au moyen de sérum, de collagène, de polylysine ou de fibronectine, de préférence de sérum de veau fœtal ou de sérum de veau nouveau-né ou de fibronectine.

13. Procédé d'obtention d'une lignée cellulaire utilisant le milieu de culture selon l'une des revendications 1 à 9, caractérisé en ce qu'il comprend essentiellement les étapes consistant à :

a) soumettre à digestion enzymatique le produit cellulaire d'une culture antérieure, notamment primaire, effectuée sur un substratum et ayant de préférence atteint la confluence, pour initier une ou plusieurs culture(s) secondaire(s),

b) inoculer les cellules animales obtenues sur un ou plusieurs substratums,

c) effectuer la ou les culture(s) secondaire(s) dans le milieu de culture, et

d) répéter les étapes a) à c) autant de fois que nécessaire pour conserver la lignée cellulaire pendant le temps désiré.

14. Procédé d'obtention d'une lignée cellulaire utilisant le milieu de culture selon l'une des revendications 1 à 9, caractérisé en ce qu'il comprend essentiellement les étapes consistant à :

a) mettre des cellules provenant d'une culture antérieure en suspension dans le milieu de culture,

b) effectuer la culture des cellules dans ledit milieu de culture, et

c) répéter les étapes a) et b) autant de fois que nécessaire pour conserver la lignée cellulaire pendant le temps désiré.

**Claims**

1. Serum-free animal cell culture medium, characterized in that it consists of :

a) a synthetic basal medium designed for animal cell culture ;

b) from 0.05 to 30 μeq/l of a mixture of the six fatty acids : palmitic acid, cis-palmitoleic acid, stearic acid, cis-oleic acid, cis-linoleic acid and cis-linolenic acid, each present under the form of the free acid or of one of its esters, in the following proportions, with respect to the free fatty acid :

— from 0.0155 to 24.0 μmole/l of palmitic acid ;
— from 0.0058 to 9.0 μmole/l of cis-palmitoleic acid ;
— from 0.0014 to 2.0 μmole/l of stearic acid ;
— from 0.0067 to 10.0 μmole/l of cis-oleic acid ;
— from 0.0178 to 27.0 μmole/l of cis-linoleic acid ; and
— from 0.0028 to 4.0 μmole/l of cis-linolenic acid,

absorbed on

c) at least one lipophile biopolymer present in an amount capable of ensuring the dissolution of the fatty acids or of their esters present in the medium.

2. Culture medium according to claim 1, characterized in that the lipophile biopolymer consists in lipid-free or not lipid-free albumin, preferably in an amount of 2 to 6 g/l of culture medium or by a dextran, preferably in an amount from 0.5 to 200 mg/l of culture medium.

3. Serum-free animal cell culture medium, characterized in that it consists of :

a) a synthetic basal medium designed for animal cell culture ;

b) from 0.05 to 30 μeq/l of a mixture of the six fatty acids : palmitic acid, cis-palmitoleic acid, stearic acid, cis-oleic acid, cis-linoleic acid and cis-linolenic acid, each present under the form of the free acid or of one of its esters, in the following proportions, with respect to the free fatty acid :

— from 0.0155 to 24.0 μmole/l of palmitic acid ;
— from 0.0058 to 9.0 μmole/l of cis-palmitoleic acid ;
— from 0.0014 to 2.0 μmole/l of stearic acid ;
— from 0.0067 to 10.0 μmole/l of cis-oleic acid ;
— from 0.0178 to 27.0 μmole/l of cis-linoleic acid ; and
— from 0.0028 to 4.0 μmole/l of cis-linolenic acid,

absorbed on

c) at least one dextran present in an amount capable of ensuring the dissolution of the fatty acids or of their esters present in the medium ; and possibly of hormones and/or growth factors.

4. Culture medium according to claim 3, characterized in that it contains from 0.5 to 200 mg/l of dextran.

5. Culture medium according to claim 3 or 4, characterized in that the dextran used is the homogeneous dextran with a molecular weight of 2 000 000.

6. Culture medium according to anyone of claims 1 to 5, characterized in that it further comprises a concentration non-cytotoxic throughout the duration of passage of the culture, of an antibiotic, preferably of wide spectrum.

7. Culture medium according to claim 6, characterized in that it comprises as antibiotic, gentamicin in the proportion of 25 to 100 mg/l.

8. Culture medium according to anyone of claims 1 to 7, characterized in that the six fatty acids are present therein in the free acid form.

9. Culture medium according to anyone of claim 1 to 8, characterized in that the synthetic basal medium consists in a medium selected from among :

— Ham media,
— Waymouth MB 752/1 medium,
— RPMI 1629 and 1640 media,
— Eagle medium,
— modified Eagle media,
— Williams E medium,
— medium 199 and the derived media of the types MEM and MEMα.

10. Process for the primary culture of animal cells using the culture medium according to anyone of claims 1 to 9, characterized in that it consists essentially of :

1) taking up the tissue containing the cells to be cultivated and mincing it very finely, in a basal culture medium, preferably in the basal medium which is to be used but not containing $Ca^{2+}$ and $Mg^{2+}$ ions, warmed (30-37 °C), under aseptic conditions,

2) after rinsing, preferably in the basal medium which is to be used but not containing $Ca^{2+}$ and $Mg^{2+}$ ions, subjecting the tissue to enzymatic digestion,

3) transferring the suspension of cells thus released into a basal medium, preferably that used for the culture, centrifuging while carrying on in parallel the digestion of the remaining tissue,

4) placing the centrifugation pellet in suspension in the culture medium containing, as the case may be, a surfactant for the culture substratum then,

5) inoculating the suspension so obtained on the culture substratum previously or simultaneously surfacted,

6) incubating in a air-tight incubator, saturated with water vapour, in the presence of a mixture of nitrogen, oxygen and carbon dioxide in proportions useable in biology of cell respiration, while renewing the culture medium periodically,

the steps 3) to 6) being repeated on the different fractions of dissociated desired cells, obtained successively in step 2), using each time a new culture substratum.

11. Process of primary culture according to claim 10, characterized in that the enzymatic digestion is carried out by means of collagenase or trypsin, preferably trypsin.

12. Process of primary culture according to claim 10 or 11, characterized in that the surfacting of the substratum is carried out by means of serum, collagen, polylysine or fibronectin, preferably fetal calf serum or newborn calf serum or fibronectin.

13. Process for obtaining a cell line using the culture medium according to anyone of claims 1 to 9, characterized in that it comprises essentially the steps consisting of :

a) subjecting to enzymatic digestion the cellular product of a prior culture, particularly a primary one, carried out on a substratum and having preferably reached confluency, to initiate one or several secondary culture(s).

b) inoculating the animal cells obtained, on one or several substrata,

c) carrying out the one or more secondary culture(s) in the culture medium, and

d) repeating steps a) to c) as many times as is necessary to preserve the cell line for the desired time.

14. Process for obtaining a cell line using the culture medium according to anyone of claims 1 to 9, characterized in that it comprises essentially the steps consisting of :

a) suspending cells of a prior culture in the culture medium,

b) carrying out the culture of the cells in said culture medium, and

c) repeating steps a) and b) as many times as necessary to preserve the cell line for the desired time.

**Patentansprüche**

1. Serumfreies Tierzellkulturmedium, dadurch gekennzeichnet, daß es zusammengesetzt ist aus :

a) einem synthetischen Basismedium, angelegt für die Terzellkultur ;

b) 0,05 bis 30 µäq/l einer Mischung aus den sechs Fettsäuren : Palmitinsäure, cis-Palmitoleinsäure, Stearinsäure, cis-Oleinsäure, cis-Linolsäure und cis-Linolensäure, wobei diese Säuren in Form der freien Säure oder eines ihrer Ester vorliegen, in folgenden Menge, bezogen auf die freie Fettsäure :

0,0155 bis 24,0 µmol/l Palmitinsäure ;
0,0058 bis 9,0 µmol/l cis-Palmitoleinsäure ;
0,0014 bis 2,0 µmol/l Stearinsäure ;
0,0067 bis 10,0 µmol/l cis-Oleinsäure ;
0,0178 bis 27,0 µmol/l cis-Linolsäure ; und
0,0028 bis 4,0 µmol/l cis-Linolensäure,

adsorbiert von

c) mindestens einem lipophilen Biopolymer, vorhanden in einer Menge, die ausreicht, die Auflösung der Fettsäuren oder ihrer Ester in dem Kulturmedium sicherzustellen.

2. Kulturmedium nach Anspruch 1, dadurch gekennzeichnet, daß das lipophile Biopolymer aus gebenenfalls delipidiertem Albumin, vorzugsweise in einer Menge von 2 bis 6 g/l des Kulturmediums, oder aus einem Dextran, vorzugsweise in einer Menge von 0,5 bis 200 mg/l des Kulturmediums besteht.

3. Serumfreies Tierzellkulturmedium, dadurch gekennzeichnet, daß es besteht aus :

a) einem synthetischen Basismedium, angelegt für die Tierzellkultur ;

b) 0,05 bis 30 µäq/l einer Mischung von den Sechs Fettsäuren : Palmitinsäure, cis-Palmitoleinsäure, Stearinsäure, cis-Oleinsäure, cis-Linolsäure und cis-Linolensäure, wobei jede Säure in Form der freien Säure oder in Form eines ihrer Ester vorliegt, in folgenden Mengen, bezogen auf die freie Säure :

0,0155 bis 24,0 µmol/l Palmitinsäure ;
0,0058 bis 9,0 µmol/l cis-Palmitoleinsäure ;
0,0014 bis 2,0 µmol/l Stearinsäure ;
0,0067 bis 10,0 µmol/l cis-Oleinsäure ;
0,0178 bis 27,0 µmol/l cis-Linolsäure ; und
0,0028 bis 4,0 µmol/l cis-Linolensäure,

adsorbiert von

c) mindestens einem Dextran, vorhanden in einer ausreichenden Menge, um die Auflösung der Fettsäuren oder ihrer Ester in dem Kulturmedium sicherzustellen ; und ggf. in Gegenwart von Hormonen und/oder Wachstumsfaktoren.

4. Kulturmedium nach Anspruch 3, dadurch gekennzeichnet, daß es 0,5 bis 200 mg/l Dextran enthält.

5. Kulturmedium nach Anspruch 3 oder 4, dadurch gekennzeichnet, daß das verwendete Dextran ein homogenes Dextran mit einem Molekulargewicht von 2 Millionen ist.

6. Kulturmedium nach einem der Ansprüche 1 bis 5, dadurch gekennzeichnet, daß es außerdem eine Konzentration eines für die gesamte Zeitspanne der Kulturpassage nicht cytotoxischen Antibiotikums, vorzugsweise eines Breitspektrumantibiotikums enthält.

7. Kulturmedium nach Anspruch 6, dadurch gekennzeichnet, daß es Gentamycin als Antibiotikum in einer Menge von 25 bis 100 mg/l enthält.

8. Kulturmedium nach einem der Ansprüche 1 bis 7, dadurch gekennzeichnet, daß die sechs Fettsäuren im Zustand der freien Säuren vorliegen.

9. Kulturmedium nach einem der Ansprüche 1 bis 8, dadurch gekennzeichnet, daß das synthetische Basismedium aus einem der folgenden Kulturmedien besteht :

Ham-Medien,
Waymouth MB 752/1-medium,
RPMI 1629 und 1640-Medien,
Eagle-Medium,
modifizierte Eagle-Medien,
Williams-Medium E,
Medium 199 und Medien, von Typ MEM und MEMα, die davon abgeleitet sind.

10. Verfahren zum Herstellen der Primärkultur tierischer Zellen unter Verwendung des Kulturmediums nach einem der Ansprüche 1 bis 9, dadurch gekennzeichnet, daß es im wesentlichen aus folgenden Stufen besteht :

1) Entnehmen des die zu bearbeitenden Zellen enthaltenden Gewebes und sehr feines Zerlegen, in einem Basiskulturmedium, vorzugsweise in dem Basismedium, das verwendet werden soll, das jedoch keine $Ca^{2+}$ und $Mg^{2+}$-Ionen enthält, das (auf 30-37 °C) aufgewärmt ist, unter aseptischen Bedingungen ;

2) nach dem Abspülen, vorzugsweise in dem Basismedium, das verwendet werden soll, das jedoch keine $Ca^{2+}$ und $Mg^{2+}$ Ionen enthält, Unterziehen des Gewebes einer enzymatischen Verdauung ;

3) Überführen der Suspension der solchermaßen freigesetzten Zellen in ein Basismedium, das vorzugsweise für die Kultur verwendet wird, und Zentrifugieren, indem man parallel dazu die Verdauung des verbleibenden Gewebes fortsetzt ;

4) Überführen des Zentrifugates in das Kulturmedium in Suspension, das ggf. ein Mittel für die Beschichtung des Kultursubstrates enthält ;

5) Inokulieren der so erhaltenen Suspension auf das vorher oder simultan beschichtete Kultursubstrat ;

6) Inkubieren in einem dichten Brutapparat, gesättigt mit Wasserdampf, in Gegenwart einer Mischung aus Stickstoff, Sauerstoff und Kohlendioxid in Mengenverhältnissen, die bei der Beatmung von Zellen in der Biologie geeignet sind, indem man periodisch das Kulturmedium erneuert,

wobei die Stufen 3) bis 6) mit den verschiedenen, nach der Stufe 2) erhaltenen disoziierten gewünschten Zellfraktionen, zu wiederholen sind, indem man jedesmal ein neues Kultursubstrat verwendet.

11. Verfahren zum Herstellen einer Primärkultur nach Anspruch 10, dadurch gekennzeichnet, daß die enzymatische Digestion mit Hilfe von Collagenase oder Trypsin, vorzugsweise mit Trypsin bewerkstelligt wird.

12. Verfahren zum Herstellen einer Primärkultur nach Anspruch 10 oder 11, dadurch gekennzeichnet, daß die Beschichtung des Substrates bewerkstelligt wird mit Hilfe eines Serums, mit Collagen, mit

Polylysin oder Fibronectin, vorzugsweise mit einem Serum eines Kalbsfötus oder mit dem Serum eines frisch geborenen Kalbes oder mit Fibronectin.

13. Verfahren zum Herstellen einer Zellinie unter Verwendung des Kulturmediums nach einem der Ansprüche 1 bis 9, dadurch gekennzeichnet, daß es im wesentlichen aus folgenden Stufen besteht :

a) Das Zellprodukt einer vorhergehenden Kultur, insbesondere einer Primärkultur, die auf ein Substrat gemacht wurde und vorzugsweise die Konfluenz erreicht hat, unterwirft man einer enzymatischen Digestion, um eine oder mehrere Sekundärkulturen zu inizijieren ;

b) die erhaltenen Tierzellen werden auf ein oder auf mehrere Substrate inokuliert ;

c) die Sekundärkultur oder die Sekundärkulturen werden in dem Kulturmedium durchgeführt, und

d) die Stufen a) bis c) werden sooft es notwendig ist, wiederholt, um die Zellinie während der gewünschten Zeit zu konservieren.

14. Verfahren zum Herstellen einer Zellinie unter Verwendung des Kulturmediums nach einem der Ansprüche 1 bis 9, dadurch gekennzeichnet, daß es im wesentlichen aus folgenden Stufen besteht :

a) Die aus einer vorhergehenden Kultur herstammenden Zellen bringt man in das Kulturmedium in Suspension,

b) die Kultur der Zellen wird in dem besagten Kulturmedium durchgeführt, und

c) die Stufen a) und b) werden, soft es notwendig ist, wiederholt, um die Zellinie während der gewünschten Zeit zu konservieren.

FIGURE 1

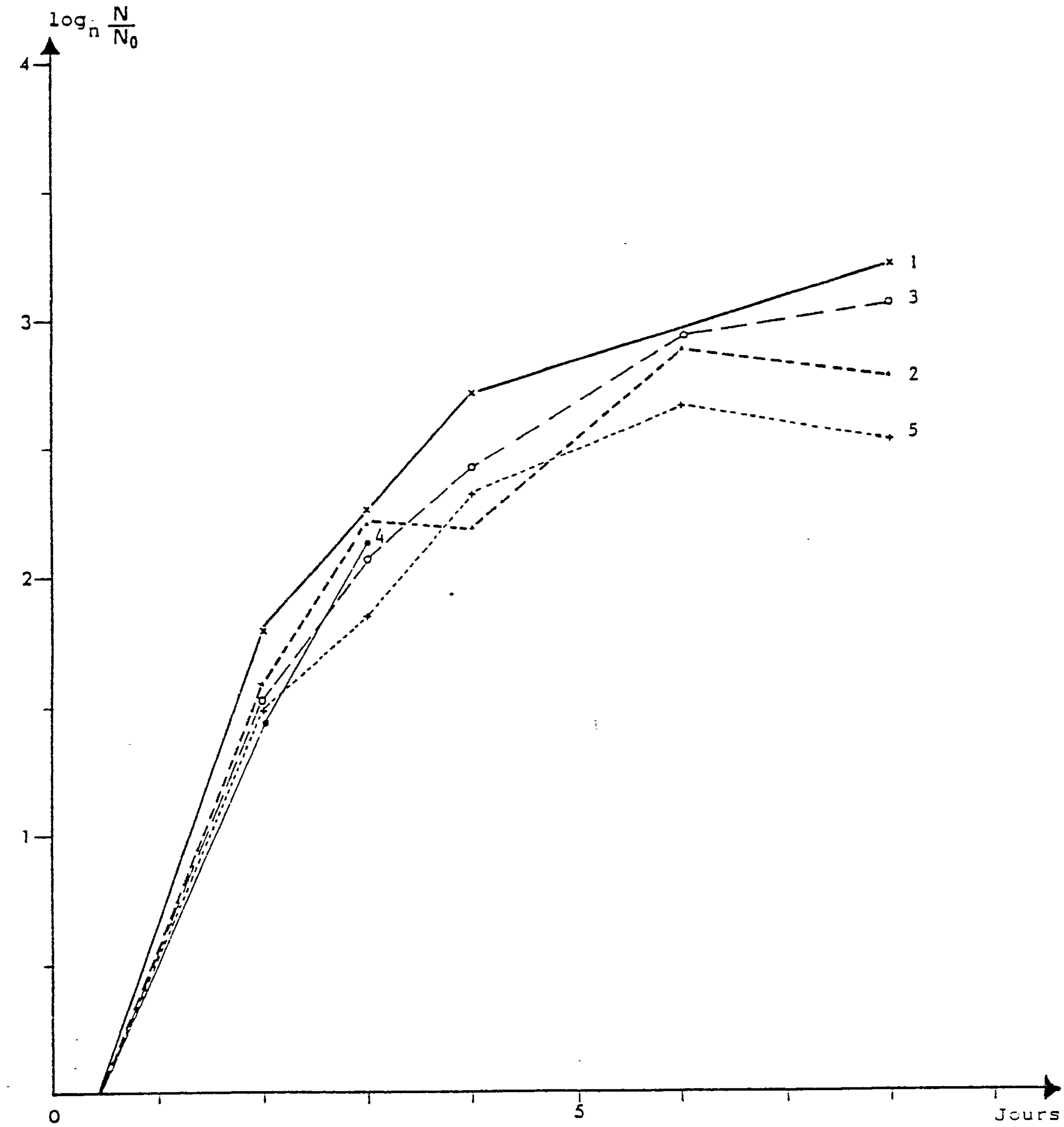

FIGURE 2

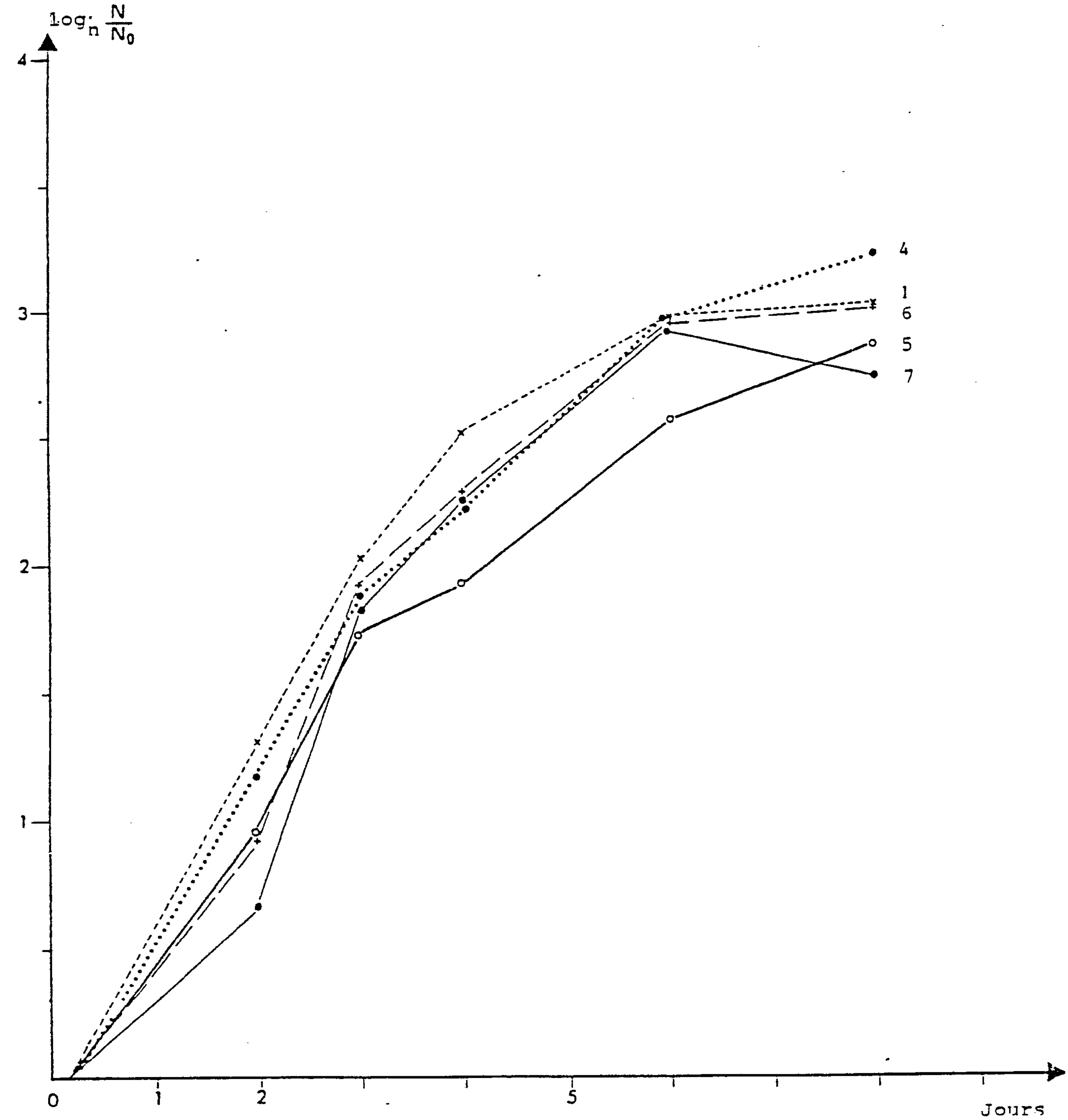

FIGURE 3

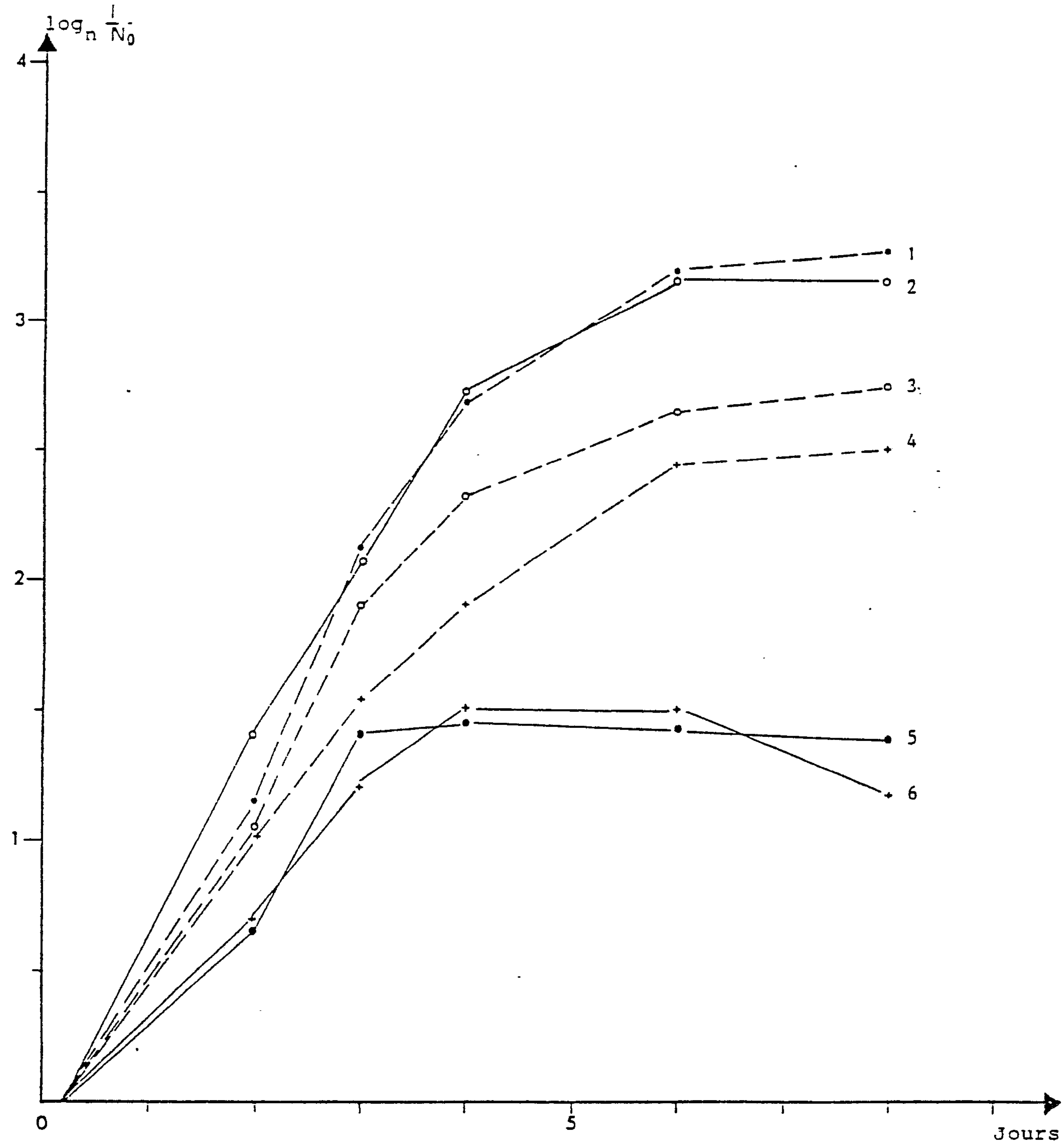

FIGURE 4